# EUROPEAN PATENT SPECIFICATION

(11) **EP 1 268 751 B1**
(45) Date of publication and mention of the grant of the patent: **17.03.2010**
(21) Application number: 00990027.5
(22) Date of filing: 28.12.2000
(51) Int. Cl.: C12N 9/00

(54) **XYLOSYLTRANSFERASE AND ISOFORMS THEREOF**
XYLOSYLTRANSFERASE UND ISOFORMEN
XYLOSYLTRANSFERASE ET ISOFORMES DE CELLE-CI

(30) Priority: 30.12.1999 EP 99126194; 01.12.2000 EP 00126233
(43) Date of publication of application: 02.01.2003
(73) Proprietor: Kleesiek, Knut, 32545 Bad Oeynhausen (DE)
(72) Inventor: KLEESIEK, Knut, 32545 Bad Oeynhausen (DE); BRINKMANN, Thomas, 33739 Bielefeld (DE); GOETTING, Christian, 32584 Loehne (DE); KUHN, Joachim, 66822 Lebach (DE)
(74) Representative: Benz, Jürgen
(86) International application number: PCT/EP2000/013311
(87) International publication number: WO 2001/049831

(56) References cited:
- GOETTING CHRISTIAN ET AL: "Xylosylation of alternatively spliced isoforms of Alzheimer APP by xylosyltransferase." JOURNAL OF PROTEIN CHEMISTRY, vol. 17, no. 3, April 1998 (1998-04), pages 295-302, XP000996086 ISSN: 0277-8033
- GOETTING CHRISTIAN ET AL: "Serum xylosyltransferase: A new biochemical marker of the sclerotic process in systemic sclerosis." JOURNAL OF INVESTIGATIVE DERMATOLOGY, vol. 112, no. 6, June 1999 (1999-06), pages 919-924, XP002165496 ISSN: 0022-202X cited in the application
- GOETTING CHRISTIAN ET AL: "Molecular cloning and expression of human UDP-D-xylose:proteoglycan core protein beta-D-xylosyltransferase and its first isoform XT-II." JOURNAL OF MOLECULAR BIOLOGY, vol. 304, no. 4, 8 December 2000 (2000-12-08), pages 517-528, XP002165497 ISSN: 0022-2836
- DATABASE EMBL [Online] EBI; Accession No: AC004707, 21 May 1998 (1998-05-21) BIRREN B. ET AL.: "Homo sapiens chromosome 17, clone hRPC.117_B_12, complete sequence." XP002173828

### SEQUENCE LISTING

<110> **Kleesiek, Knut**
<120> **Xylosyltransferase and isoforms thereof**
<130> Xylosyltransferase
<140>
   <141>
<160> 36
<170> PatentIn Ver. 2.1
<210> 1
   <211>. 3726
   <212> DNA
   <213> Homo sapiens
<220>
   <221> CDS
   <222> (1)..(2484)
   <223> human XT-I (not completely)
<220>
   <221> misc_binding
   <222> (544)..(552)
   <223> binding motif for nucleotide-sugars
<220>
   <221> misc_feature
   <222> (280)..(282)
   <223> N-glycosylation site
<220>
   <221> misc_feature
   <222> (865)..(867)
   <223> N-glycosylation site
<220>
   <221> misc_feature
   <222> (1933)..(1935)
   <223> N-glycosylation site
<400> 1
<210> 2
   <211> 827
   <212> PRT
   <213> Homo sapiens
<400> 2
   Thr Gln Asp Gly Tyr Phe Ser His Arg Pro Lys Glu Lys Val Arg Thr
    1               5                  10                  15
   Asp Ser Asn Asn Glu Asn Ser Val Pro Lys Asp Phe Glu Asn Val Asp
               20                  25                  30
   Asn Ser Asn Phe Ala Pro Arg Thr Gln Lys Gln Lys His Gln Pro Glu
           35                  40                  45
   Leu Ala Lys Lys Pro Pro Ser Arg Gln Lys Glu Leu Leu Lys Arg Lys
       50                  55                  60
   Leu Glu Gln Gln Glu Lys Gly Lys Gly His Thr Phe Pro Gly Lys Gly
   65                  70                  75                  80
   Pro Gly Glu Val Leu Pro Pro Gly Asp Arg Ala Ala Ala Asn Ser Ser
                   85                  90                  95
   His Gly Lys Asp Val Ser Arg Pro Pro His Ala Arg Lys Thr Gly Gly
               100                 105                 110
   Ser Ser Pro Glu Thr Lys Tyr Asp Gln Pro Pro Lys Cys Asp Ile Ser
           115                 120                 125
   Gly Lys Glu Ala Ile Ser Ala Leu Ser Arg Ala Lys Ser Lys His Cys
       130                 135                 140
   Arg Gln Glu Ile Gly Glu Thr Tyr Cys Arg His Lys Leu Gly Leu Leu
   145                 150                 155                 160
   Met Pro Glu Lys Val Thr Arg Phe Cys Pro Leu Glu Gly Lys Ala Asn
                   165                 170                 175
   Lys Asn Val Gln Trp Asp Glu Asp Ser Val Glu Tyr Met Pro Ala Asn
   180 185 190
   Pro Val Arg Ile Ala Phe Val Leu Val Val His Gly Arg Ala Ser Arg
   195 200 205
   Gln Leu Gln Arg Met Phe Lys Ala Ile Tyr His Lys Asp His Phe Tyr
   210 215 220
   Tyr Ile His Val Asp Lys Arg Ser Asn Tyr Leu His Arg Gln Val Leu
   225 230 235 240
   Gln Val Ser Arg Gln Tyr Ser Asn Val Arg Val Thr Pro Trp Arg Met
   245 250 255
   Ala Thr Ile Trp Gly Gly Ala Ser Leu Leu Ser Thr Tyr Leu Gln Ser
   260 265 270
   Met Arg Asp Leu Leu Glu Met Thr Asp Trp Pro Trp Asp Phe Phe Ile
   275 280 285
   Asn Leu Ser Ala Ala Asp Tyr Pro Ile Arg Thr Asn Asp Gln Leu Val
   290 295 300
   Ala Phe Leu Ser Arg Tyr Arg Asp Met Asn Phe Leu Lys Ser His Gly
   305 310 315 320
   Arg Asp Asn Ala Arg Phe Ile Arg Lys Gln Gly Leu Asp Arg Leu Phe
   325 330 335
   Leu Glu Cys Asp Ala His Met Trp Arg Leu Gly Asp Arg Arg Ile Pro
   340 345 350
   Glu Gly Ile Ala Val Asp Gly Gly Ser Asp Trp Phe Leu Leu Asn Arg
   355 360 365
   Arg Phe Val Glu Tyr Val Thr Phe Ser Thr Asp Asp Leu Val Thr Lys
   370 375 380
   Met Lys Gln Phe Tyr Ser Tyr Thr Leu Leu Pro Ala Glu Ser Phe Phe
   385 390 395 400
   His Thr Val Leu Glu Asn Ser Pro His Cys Asp Thr Met Val Asp Asn
   405 410 415
   Asn Leu Arg Ile Thr Asn Trp Asn Arg Lys Leu Gly Cys Lys Cys Gln
   420 425 430
   Tyr Lys His Ile Val Asp Trp Cys Gly Cys Ser Pro Asn Asp Phe Lys
   435 440 445
   Pro Gln Asp Phe His Arg Phe Gln Gln Thr Ala Arg Pro Thr Phe Phe
   450 455 460
   Ala Arg Lys Phe Glu Ala Val Val Asn Gln Glu Ile Ile Gly Gln Leu
   465 470 475 480
   Asp Tyr Tyr Leu Tyr Gly Asn Tyr Pro Ala Gly Thr Pro Gly Leu Arg
   485 490 495
   Ser Tyr Trp Glu Asn Val Tyr Asp Glu Pro Asp Gly Ile His Ser Leu
   500 505 510
   Ser Asp Val Thr Leu Thr Leu Tyr His Ser Phe Ala Arg Leu Gly Leu
   515 520 525
   Arg Arg Ala Glu Thr Ser Leu His Thr Asp Gly Glu Asn Ser Cys Arg
   530 535 540
   Tyr Tyr Pro Met Gly His Pro Ala Ser Val His Leu Tyr Phe Leu Ala
   545 550 555 560
   Asp Arg Phe Gln Gly Phe Leu Ile Lys His His Ala Thr Asn Leu Ala
   565 570 575
   Val Ser Lys Leu Glu Thr Leu Glu Thr Trp Val Met Pro Lys Lys Val
   580 585 590
   Phe Lys Ile Ala Ser Pro Pro Ser Asp Phe Gly Arg Leu Gln Phe Ser
   595 600 605
   Glu Val Gly Thr Asp Trp Asp Ala Lys Glu Arg Leu Phe Arg Asn Phe
   610 615 620
   Gly Gly Leu Leu Gly Pro Met Asp Glu Pro Val Gly Met Gln Lys Trp
   625 630 635 640
   Gly Lys Gly Pro Asn Val Thr Val Thr Val Ile Trp Val Asp Pro Val
   645 650 655
   Asn Val Ile Ala Ala Thr Tyr Asp Ile Leu Ile Glu Ser Thr Ala Glu
   660 665 670
   Phe Thr His Tyr Lys Pro Pro Leu Asn Leu Pro Leu Arg Pro Gly Val
   675 680 685
   Trp Thr Val Lys Ile Leu His His Trp Val Pro Val Ala Glu Thr Lys
   690 695 700
   Phe Leu Val Ala Pro Leu Thr Phe Ser Asn Arg Gln Pro Ile Lys Pro
   705 710 715 720
   Glu Glu Ala Leu Lys Leu His Asn Gly Pro Leu Arg Asn Ala Tyr Met
   725 730 735
   Glu Gln Ser Phe Gln Ser Leu Asn Pro Val Leu Ser Leu Pro Ile Asn
   740 745 750
   Pro Ala Gln Val Glu Gln Ala Arg Arg Asn Ala Ala Ser Thr Gly Thr
   755 760 765
   Ala Leu Glu Gly Trp Leu Asp Ser Leu Val Gly Gly Met Trp Thr Ala
   770 775 780
   Met Asp Ile Cys Ala Thr Gly Pro Thr Ala Cys Pro Val Met Gln Thr
   785 790 795 800
   Cys Ser Gln Thr Ala Trp Ser Ser Phe Ser Pro Asp Pro Lys Ser Glu
   805 810 815
   Leu Gly Ala Val Lys Pro Asp Gly Arg Leu Arg
   820 825
<210> 3
   <211> 3608
   <212> DNA
   <213> Homo sapiens
<220>
   <221> CDS
   <222> (150)..(2744)
   <223> human XT-II
<220>
   <221> misc_feature
   <222> (513)..(515)
   <223> N-glycosylation site
<220>
   <221> misc_feature
   <222> (1128)..(1130)
   <223> N-glcosylation site
<220>
   <221> misc_feature
   <222> (2196)..(2198)
   <223> N-glycosylation site
<400> 3
   cgcaggcccc gcccccggcc ccgccccggc gcgcgcccgg cccgcctccc taggcgtgga 60
   ggaggggggg cggctcagcc ccgcgcccgt gcgcgcgctc gcggccgggt tgcagggctg 120
   ggcgcgcgcc ccgcgtcccg ggcaggaag atg gtg gcg agc gcg cga gtg cag   173
                                   Met Val Ala Ser Ala Arg Val Gln
                                   1               5
   aag ctg gtg cgg cgc tac aag ctg gcg att gcc acg gcg ctg gcc atc   221
   Lys Leu Val Arg Arg Tyr Lys Leu Ala Ile Ala Thr Ala Leu Ala Ile
       10                  15                  20
   ctg ctg ctg cag ggc ctg gta gtg tgg agc ttc agc ggc ctg gag gag   269
   Leu Leu Leu Gln Gly Leu Val Val Trp Ser Phe Ser Gly Leu Glu Glu
   25                  30                  35                  40
   gac gag gcg ggc gag aaa gga agg cag agg aag cca cgg cca ctg gac   317
   Asp Glu Ala Gly Glu Lys Gly Arg Gln Arg Lys Pro Arg Pro Leu Asp
                   45                  50                  55
   cct ggc gaa ggt tcc aag gac aca gac agt tca gca ggg cga cgg ggc   365
   Pro Gly Glu Gly Ser Lys Asp Thr Asp Ser Ser Ala Gly Arg Arg Gly
               60                  65                  70
   agc aca ggc aga agg cat ggg cgc tgg cgg ggc cgt gct gag agc cca   413
   Ser Thr Gly Arg Arg His Gly Arg Trp Arg Gly Arg Ala Glu Ser Pro
           75                  80                  85
   gga gtg ccc gtg gcc aag gtg gta cgg gca gta acc agc cgg cag aga   461
   Gly Val Pro Val Ala Lys Val Val Arg Ala Val Thr Ser Arg Gln Arg
       90                  95                  100
   gcc agc cgg cgg gtc cca cct gcc cca cct ccg gaa gcc cca ggc cgc   509
   Ala Ser Arg Arg Val Pro Pro Ala Pro Pro Pro Glu Ala Pro Gly Arg
   105                 110                 115                 120
   cag aac ctg agt ggg gca gca gct ggg gag gcg ctg gta ggg gca gct   557
   Gln Asn Leu Ser Gly Ala Ala Ala Gly Glu Ala Leu Val Gly Ala Ala
                   125                 130                 135
   ggc ttc cca cca cac gga gat aca ggg agc gtg gag ggc gcc ccc cag     605
   Gly Phe Pro Pro His Gly Asp Thr Gly Ser Val Glu Gly Ala Pro Gln
               140                 145                 150
   ccc acg gac aat ggc ttc acc ccc aag tgc gag atc gtg ggc aag gac     653
   Pro Thr Asp Asn Gly Phe Thr Pro Lys Cys Glu Ile Val Gly Lys Asp
           155                 160                 165
   gca ctg tct gca ctg gcc cgg gcc agc acc aag cag tgc cag cag gag     701
   Ala Leu Ser Ala Leu Ala Arg Ala Ser Thr Lys Gln Cys Gln Gln Glu
       170                 175                 180
   atc gcc aat gtg gtg tgc ctg cac cag gct ggg agc ctc atg ccc aag     749
   Ile Ala Asn Val Val Cys Leu His Gln Ala Gly Ser Leu Met Pro Lys
   185                 190                 195                 200
   gct gtg ccc cgg cac tgt cag ctg act ggg aag atg agc ccc ggc atc     797
   Ala Val Pro Arg His Cys Gln Leu Thr Gly Lys Met Ser Pro Gly Ile
                   205                 210                 215
   cag tgg gat gag agc caa gcc cag cag ccc atg gat ggc ccc ccg gtg     845
   Gln Trp Asp Glu Ser Gln Ala Gln Gln Pro Met Asp Gly Pro Pro Val
               220                 225                 230
   cga atc gcc tac atg ctg gtg gtt cac ggc cgc gcc atc cgc cag ctg     893
   Arg Ile Ala Tyr Met Leu Val Val His Gly Arg Ala Ile Arg Gln Leu
           235                 240                 245
   aag cgt ctc ctc aag gcc gtt tat cac gag cag cac ttc ttt tac atc     941
   Lys Arg Leu Leu Lys Ala Val Tyr His Glu Gln His Phe Phe Tyr Ile
       250                 255                 260
   cat gtg gac aag cgt tcc gac tac ctg cac cgg gag gtg gtg gag ctg     989
   His Val Asp Lys Arg Ser Asp Tyr Leu His Arg Glu Val Val Glu Leu
   265                 270                 275                 280
   gcc cag ggc tat gat aac gtg cgg gtg acg ccc tgg cgc atg gtt acc     1037
   Ala Gln Gly Tyr Asp Asn Val Arg Val Thr Pro Trp Arg Met Val Thr
                   285                 290                 295
   atc tgg ggc ggg gcc agc ctc ctg acg atg tac ctg cgg agc atg cgg     1085
   Ile Trp Gly Gly Ala Ser Leu Leu Thr Met Tyr Leu Arg Ser Met Arg
               300                 305                 310
   gac ctg cta gag gtg cct ggc tgg gcc tgg gac ttc ttc atc aac ctc     1133
   Asp Leu Leu Glu Val Pro Gly Trp Ala Trp Asp Phe Phe Ile Asn Leu
           315                 320                 325
   agt gcc act gac tat cca acc agg acc aat gag gag ctg gtg gca ttc     1181
   Ser Ala Thr Asp Tyr Pro Thr Arg Thr Asn Glu Glu Leu Val Ala Phe
       330                 335                 340
   cta tcc aag aac cgg gac aag aat ttc ctc aag tca cat ggc cgg gac     1229
   Leu Ser Lys Asn Arg Asp Lys Asn Phe Leu Lys Ser His Gly Arg Asp
   345                 350                 355                 360
   aac tcc agg ttc atc aag aaa cag ggc ctg gac cgg ctc ttc cat gag     1277
   Asn Ser Arg Phe Ile Lys Lys Gln Gly Leu Asp Arg Leu Phe His Glu
                   365                 370                 375
   tgc gac tca cac atg tgg cgc ctg ggc gag cgg cag atc cca gca ggc     1325
   Cys Asp Ser His Met Trp Arg Leu Gly Glu Arg Gln Ile Pro Ala Gly
               380                 385                 390
   att gtg gtg gat ggc ggt tct gac tgg ttc gtg ctg aca cgc agc ttt    1373
   Ile Val Val Asp Gly Gly Ser Asp Trp Phe Val Leu Thr Arg Ser Phe
           395                 400                 405
   gtg gag tat gtg gtg tac aca gat gac ccg ctt gtg gcc cag ctg cgc    1421
   Val Glu Tyr Val Val Tyr Thr Asp Asp Pro Leu Val Ala Gln Leu Arg
       410                 415                 420
   cag ttc tac aca tac aca ctg ctc cca gcc gag tcc ttc ttc cac acg    1469
   Gln Phe Tyr Thr Tyr Thr Leu Leu Pro Ala Glu Ser Phe Phe His Thr
   425                 430                 435                 440
   gtg ctg gag aac agc ctg gcc tgt gag acc ctc gtg gac aac aac ctg    1517
   Val Leu Glu Asn Ser Leu Ala Cys Glu Thr Leu Val Asp Asn Asn Leu
                   445                 450                 455
   cgg gtc acc aac tgg aac cgc aag ctg ggc tgc aag tgc cag tac aag    1565
   Arg Val Thr Asn Trp Asn Arg Lys Leu Gly Cys Lys Cys Gln Tyr Lys
               460                 465                 470
   cac att gtg gac tgg tgt ggc tgc tcc ccc aac gac ttc aag cca cag    1613
   His Ile Val Asp Trp Cys Gly Cys Ser Pro Asn Asp Phe Lys Pro Gln
           475                 480                 485
   gac ttc ctc cgg ctg cag caa gtc tcc aga ccc acc ttc ttc gcc cgg    1661
   Asp Phe Leu Arg Leu Gln Gln Val Ser Arg Pro Thr Phe Phe Ala Arg
       490                 495                 500
   aag ttc gag tcg act gtg aac cag gag gtg ctg gaa atc ctg gac ttc    1709
   Lys Phe Glu Ser Thr Val Asn Gln Glu Val Leu Glu Ile Leu Asp Phe
   505                 510                 515                 520
   cac ctg tac ggc agc tac ccc ccc ggc acg cca gcc ctc aag gcc tac    1757
   His Leu Tyr Gly Ser Tyr Pro Pro Gly Thr Pro Ala Leu Lys Ala Tyr
                   525                 530                 535
   tgg gag aac acc tac gac gcg gct gat ggc ccc agt ggg ctc agt gat    1805
   Trp Glu Asn Thr Tyr Asp Ala Ala Asp Gly Pro Ser Gly Leu Ser Asp
               540                 545                 550
   gtc atg ctc act gct tac aca gcc ttc gcc cgc ctc agc ctg cac cat    1853
   Val Met Leu Thr Ala Tyr Thr Ala Phe Ala Arg Leu Ser Leu His His
           555                 560                 565
   gcc gcc act gct gca ccc cca atg ggc acc cca ctc tgc agg ttt gag    1901
   Ala Ala Thr Ala Ala Pro Pro Met Gly Thr Pro Leu Cys Arg Phe Glu
       570                 575                 580
   ccc agg ggc ttg ccg tcc agc gtg cac ctg tat ttc tat gac gac cat    1949
   Pro Arg Gly Leu Pro Ser Ser Val His Leu Tyr Phe Tyr Asp Asp His
   585                 590                 595                 600
   ttc cag ggc tac ctg gtg acg cag gcg gtg cag ccc tca gcc cag ggg    1997
   Phe Gln Gly Tyr Leu Val Thr Gln Ala Val Gln Pro Ser Ala Gln Gly
                   605                 610                 615
   ccg gca gag acg ctt gag atg tgg ctg atg ccc caa ggg tcg ctg aag    2045
   Pro Ala Glu Thr Leu Glu Met Trp Leu Met Pro Gln Gly Ser Leu Lys
               620                 625                 630
   ctg ttg ggg cgc agt gac cag gcc agc cgg ctc cag agt ctg gag gtt    2093
   Leu Leu Gly Arg Ser Asp Gln Ala Ser Arg Leu Gln Ser Leu Glu Val
           635                 640                 645
   ggc act gat tgg gac ccc aaa gag cgt ctt ttc cgg aac ttt ggg ggg   2141
   Gly Thr Asp Trp Asp Pro Lys Glu Arg Leu Phe Arg Asn Phe Gly Gly
       650                 655                 660
   tta ctg ggg ccg ctg gac gag cct gtg gcc gtg cag cgc tgg gcc cgg   2189
   Leu Leu Gly Pro Leu Asp Glu Pro Val Ala Val Gln Arg Trp Ala Arg
   665                 670                 675                 680
   ggc ccc aac ctc aca gcc aca gtg gtc tgg atc gac cca acc tat gtg   2237
   Gly Pro Asn Leu Thr Ala Thr Val Val Trp Ile Asp Pro Thr Tyr Val
                   685                 690                 695
   gtg gcc aca tct tat gac atc aca gta gat acg gag act gag gtc acg   2285
   Val Ala Thr Ser Tyr Asp Ile Thr Val Asp Thr Glu Thr Glu Val Thr
               700                 705                 710
   caa tac aag ccc cca ctg agc cgg ccc ctg cgg cca ggg ccc tgg act   2333
   Gln Tyr Lys Pro Pro Leu Ser Arg Pro Leu Arg Pro Gly Pro Trp Thr
           715                 720                 725
   gtt cga ctc ctt cag ttc tgg gaa ccg ctg ggt gag acc cgc ttc ctt   2381
   Val Arg Leu Leu Gln Phe Trp Glu Pro Leu Gly Glu Thr Arg Phe Leu
       730                 735                 740
   gtg ctg ccc ttg acc ttc aac cgc aaa cta cct ctc agg aaa gat gat   2429
   Val Leu Pro Leu Thr Phe Asn Arg Lys Leu Pro Leu Arg Lys Asp Asp
   745                 750                 755                 760
   gcc agc tgg ctg cac gca ggg cca ccc cac aac gag tac atg gag cag   2477
   Ala Ser Trp Leu His Ala Gly Pro Pro His Asn Glu Tyr Met Glu Gln
                   765                 770                 775
   agt ttc cag ggc ctg agt agc atc ctg aac ctg cct cag ccg gag ctc   2525
   Ser Phe Gln Gly Leu Ser Ser Ile Leu Asn Leu Pro Gln Pro Glu Leu
               780                 785                 790
   gcg gag gag gct gcc cag cgg cac aca cag ctc aca ggc cct gcg ctc   2573
   Ala Glu Glu Ala Ala Gln Arg His Thr Gln Leu Thr Gly Pro Ala Leu
           795                 800                 805
   gag gcc tgg aca gac agg gaa ctg agc agc ttc tgg tcc gtg gct gga   2621
   Glu Ala Trp Thr Asp Arg Glu Leu Ser Ser Phe Trp Ser Val Ala Gly
       810                 815                 820
   ctg tgt gcc ata ggc ccc tct ccc tgc ccc tcc ctg gag ccc tgc aga   2669
   Leu Cys Ala Ile Gly Pro Ser Pro Cys Pro Ser Leu Glu Pro Cys Arg
   825                 830                 835                 840
   ctg acc agc tgg agc tct ctg tcc ccc gac ccc aaa tca gag ctg ggg   2717
   Leu Thr Ser Trp Ser Ser Leu Ser Pro Asp Pro Lys Ser Glu Leu Gly
                   845                 850                 855
   cct gtc aaa gca gac ggg cga ctc agg tagcagggcc ccagccagta         2764
   Pro Val Lys Ala Asp Gly Arg Leu Arg
               860                 865
   cccgtggagg acccgggaaa ttgcacctta cagacagtgg aggggtgtcc cctcccacag 2824
   gcaagaacca gaggcccagg ctgcacaccc atttcagcca tcaagaaccc acacagacgg 2884
   cagggaaggt ggacacagta tgaactactg ctgatgtctc tgttggggat cagagggctg 2944
   gcgggaacgc gagaagggca ccagcagcat tccacaccca gctcttcctc accttcctgt 3004
   ctagtttgaa tttctttttt ttctttcttt tttttttttt tttaatttaa aaaggaaaat 3064
   gggtggttgg gagagaaact agaacagaag atgtgcaata gggctcagag cagccccagg 3124
   aagtgggcca tgtctgtggg gagcagtggc ttgctgagcc cacctgctat tgttctgcac 3184
   gaggctgggc ctgcctcact ctccagggcc tcatgcccca ttctgggcct gtggtgctcg 3244
   tggctgaggc tccacagggc tgcaagtgcc ctgccaggct ctaaggcccg aagaacaggt 3304
   gatatcgggg gcgctgcaga gctgggctct agcaggacag ttcttttgta gccaggggac 3364
   cctagaagtg gggtggggcg gctccagggc tttccagcat accctgcccc tggatgggaa 3424
   aggcagggtc agggcccact gagacccatg cagagtttcc aggtgtgcac tggaagtgag 3484
   gtcacatgag cagcgtggga agaagactct gtcaagactc tcagaagaac ctggagtaat 3544
   tgtgcctgaa gctcagcgtg aagtctgaaa tatgcaacag aagaaatata tctctatctc 3604
   tcta                                                              3608
<210> 4
   <211> 865
   <212> PRT
   <213> Homo sapiens
<400> 4
   Met Val Ala Ser Ala Arg Val Gln Lys Leu Val Arg Arg Tyr Lys Leu
   1               5                   10                  15
   Ala Ile Ala Thr Ala Leu Ala Ile Leu Leu Leu Gln Gly Leu Val Val
               20                  25                  30
   Trp Ser Phe Ser Gly Leu Glu Glu Asp Glu Ala Gly Glu Lys Gly Arg
           35                  40                  45
   Gln Arg Lys Pro Arg Pro Leu Asp Pro Gly Glu Gly Ser Lys Asp Thr
       50                  55                  60
   Asp Ser Ser Ala Gly Arg Arg Gly Ser Thr Gly Arg Arg His Gly Arg
   65                  70                  75                  80
   Trp Arg Gly Arg Ala Glu Ser Pro Gly Val Pro Val Ala Lys Val Val
                   85                  90                  95
   Arg Ala Val Thr Ser Arg Gln Arg Ala Ser Arg Arg Val Pro Pro Ala
               100                 105                 110
   Pro Pro Pro Glu Ala Pro Gly Arg Gln Asn Leu Ser Gly Ala Ala Ala
           115                 120                 125
   Gly Glu Ala Leu Val Gly Ala Ala Gly Phe Pro Pro His Gly Asp Thr
       130                 135                 140
   Gly Ser Val Glu Gly Ala Pro Gln Pro Thr Asp Asn Gly Phe Thr Pro
   145                 150                 155                 160
   Lys Cys Glu Ile Val Gly Lys Asp Ala Leu Ser Ala Leu Ala Arg Ala
                   165                 170                 175
   Ser Thr Lys Gln Cys Gln Gln Glu Ile Ala Asn Val Val Cys Leu His
               180                 185                 190
   Gln Ala Gly Ser Leu Met Pro Lys Ala Val Pro Arg His Cys Gln Leu
           195                 200                 205
   Thr Gly Lys Met Ser Pro Gly Ile Gln Trp Asp Glu Ser Gln Ala Gln
       210                 215                 220
   Gln Pro Met Asp Gly Pro Pro Val Arg Ile Ala Tyr Met Leu Val Val
   225                 230                 235                 240
   His Gly Arg Ala Ile Arg Gln Leu Lys Arg Leu Leu Lys Ala Val Tyr
                   245                 250                 255
   His Glu Gln His Phe Phe Tyr Ile His Val Asp Lys Arg Ser Asp Tyr
               260                     265                     270
   Leu His Arg Glu Val Val Glu Leu Ala Gln Gly Tyr Asp Asn Val Arg
           275                     280                     285
   Val Thr Pro Trp Arg Met Val Thr Ile Trp Gly Gly Ala Ser Leu Leu
       290                     295                     300
   Thr Met Tyr Leu Arg Ser Met Arg Asp Leu Leu Glu Val Pro Gly Trp
   305                     310                     315                 320
   Ala Trp Asp Phe Phe Ile Asn Leu Ser Ala Thr Asp Tyr Pro Thr Arg
                   325                     330                     335
   Thr Asn Glu Glu Leu Val Ala Phe Leu Ser Lys Asn Arg Asp Lys Asn
               340                     345                     350
   Phe Leu Lys Ser His Gly Arg Asp Asn Ser Arg Phe Ile Lys Lys Gln
           355                     360                     365
   Gly Leu Asp Arg Leu Phe His Glu Cys Asp Ser His Met Trp Arg Leu
       370                     375                     380
   Gly Glu Arg Gln Ile Pro Ala Gly Ile Val Val Asp Gly Gly Ser Asp
   385                     390                     395                 400
   Trp Phe Val Leu Thr Arg Ser Phe Val Glu Tyr Val Val Tyr Thr Asp
                   405                     410                     415
   Asp Pro Leu Val Ala Gln Leu Arg Gln Phe Tyr Thr Tyr Thr Leu Leu
               420                     425                     430
   Pro Ala Glu Ser Phe Phe His Thr Val Leu Glu Asn Ser Leu Ala Cys
           435                     440                     445
   Glu Thr Leu Val Asp Asn Asn Leu Arg Val Thr Asn Trp Asn Arg Lys
       450                     455                     460
   Leu Gly Cys Lys Cys Gln Tyr Lys His Ile Val Asp Trp Cys Gly Cys
   465                     470                     475                 480
   Ser Pro Asn Asp Phe Lys Pro Gln Asp Phe Leu Arg Leu Gln Gln Val
                   485                     490                     495
   Ser Arg Pro Thr Phe Phe Ala Arg Lys Phe Glu Ser Thr Val Asn Gln
               500                     505                     510
   Glu Val Leu Glu Ile Leu Asp Phe His Leu Tyr Gly Ser Tyr Pro Pro
           515                     520                     525
   Gly Thr Pro Ala Leu Lys Ala Tyr Trp Glu Asn Thr Tyr Asp Ala Ala
       530                     535                     540
   Asp Gly Pro Ser Gly Leu Ser Asp Val Met Leu Thr Ala Tyr Thr Ala
   545                     550                     555                 560
   Phe Ala Arg Leu Ser Leu His His Ala Ala Thr Ala Ala Pro Pro Met
                   565                     570                     575
   Gly Thr Pro Leu Cys Arg Phe Glu Pro Arg Gly Leu Pro Ser Ser Val
               580                     585                     590
   His Leu Tyr Phe Tyr Asp Asp His Phe Gln Gly Tyr Leu Val Thr Gln
   595 600 605
   Ala Val Gln Pro Ser Ala Gln Gly Pro Ala Glu Thr Leu Glu Met Trp
   610 615 620
   Leu Met Pro Gln Gly Ser Leu Lys Leu Leu Gly Arg Ser Asp Gln Ala
   625 630 635 640
   Ser Arg Leu Gln Ser Leu Glu Val Gly Thr Asp Trp Asp Pro Lys Glu
   645 650 655
   Arg Leu Phe Arg Asn Phe Gly Gly Leu Leu Gly Pro Leu Asp Glu Pro
   660 665 670
   Val Ala Val Gln Arg Trp Ala Arg Gly Pro Asn Leu Thr Ala Thr Val
   675 680 685
   Val Trp Ile Asp Pro Thr Tyr Val Val Ala Thr Ser Tyr Asp Ile Thr
   690 695 700
   Val Asp Thr Glu Thr Glu Val Thr Gln Tyr Lys Pro Pro Leu Ser Arg
   705 710 715 720
   Pro Leu Arg Pro Gly Pro Trp Thr Val Arg Leu Leu Gln Phe Trp Glu
   725 730 735
   Pro Leu Gly Glu Thr Arg Phe Leu Val Leu Pro Leu Thr Phe Asn Arg
   740 745 750
   Lys Leu Pro Leu Arg Lys Asp Asp Ala Ser Trp Leu His Ala Gly Pro
   755 760 765
   Pro His Asn Glu Tyr Met Glu Gln Ser Phe Gln Gly Leu Ser Ser Ile
   770 775 780
   Leu Asn Leu Pro Gln Pro Glu Leu Ala Glu Glu Ala Ala Gln Arg His
   785 790 795 800
   Thr Gln Leu Thr Gly Pro Ala Leu Glu Ala Trp Thr Asp Arg Glu Leu
   805 810 815
   Ser Ser Phe Trp Ser Val Ala Gly Leu Cys Ala Ile Gly Pro Ser Pro
   820 825 830
   Cys Pro Ser Leu Glu Pro Cys Arg Leu Thr Ser Trp Ser Ser Leu Ser
   835 840 845
   Pro Asp Pro Lys Ser Glu Leu Gly Pro Val Lys Ala Asp Gly Arg Leu
   850 855 860
   Arg
   865
<210> 9
   <211> 17
   <212> PRT
   <213> Artificial Sequence
<220>
   <223> Description of Artificial Sequence: peptide
<400> 9
   Cys Ser Arg Gln Lys Glu Leu Leu Lys Arg Lys Leu Glu Gln Gln Glu
   1 5 10 15
   Lys
<210> 10
   <211> 11
   <212> PRT
   <213> Artificial Sequence
<220>
   <223> Description of Artificial Sequence: peptide
<400> 10
   Gln Glu Glu Glu Gly Ser Gly Gly Gly Gln Lys
   1 5 10
<210> 11
   <211> 12
   <212> PRT
   <213> Artificial Sequence
<220>
   <223> Description of Artificial Sequence: peptide
<400> 11
   Ser Glu Asn Glu Gly Ser Gly Met Ala Glu Gln Lys
   1 5 10
<210> 12
   <211> 12
   <212> PRT
   <213> Artificial Sequence
<220>
   <223> Description of Artificial Sequence: peptide
<400> 12
   Thr Glu Asn Glu Gly Ser Gly Leu Thr Asn Ile Lys
   1 5 10

## Description

The invention relates to the isolation, purification and characterization of the enzyme xylosyltransferase (defined as "**XT**"). The invention describes for the first time that XT occurs in at least two isoforms ("XT-I", "XT-II"). The invention relates furthermore to the recombinant cloning and expression of human and rat XT-I and XT-II and discloses their DNA and protein sequences. The enzymes according to the invention can be used as therapeutic agents and as diagnostic markers, e.g. for the determination of enhanced proteoglycyn biosynthesis, and as biochemical markers for determination of several pathological processes such as systemic sclerosis.

### Background of the Invention

Proteoglycans are polyanionic molecules widely expressed in animal cells and virtually every tissue. These abundant molecules are present in the extracellular matrix and on the cell surface and serve a wide range of functions. They are increasingly implicated as important regulators in many biological processes, such as extracellular matrix deposition, cell membrane signal transfer, morphogenesis, cell migration, normal and tumor cell growth and viral infection (Ruoslahti, 1989, J. Biol. Chem. 264, 13369-1372; Herold et al., 1994, J. Gen. Virol. 75, 1211-1222). Proteoglycans mediate diverse cellular processes through interaction with a variety of protein ligands. In most of these bindings electrostatic interactions with the glycosaminoglycan chains attached to the core protein are involved (Kjellen & Lindahl, 1991, Annu. Rev. Biochem. 60, 443-475). Thus, the biological activity of proteoglycans is intimately related to the glycosaminoglycan biosynthesis.

The sulfated glycosaminoglycans chondroitin sulfate, heparan sulfate, heparin and dermatan sulfate are bound to the proteoglycan core protein by a xylose-galactose-galactose binding region (Kjellen & Lindahl, *1991, l.c.*). UDP-D-xylose:proteoglycan core protein β-D-xylosyltransferase (EC 2.4.2.26) is the chain-initiating enzyme involved in the biosynthesis of glycosaminoglycan containing proteoglycans (Schwarz, 1977, J. Biol. Chem. 252, 6316-6321*;* Kearns et al., 1991, Biochemistry 30, 7477-7483*).* The enzyme catalyzes the transfer of D-xylose from UDP-D-xylose to specific serine residues of the core protein and is a regulatory factor in chondroitin sulfate biosynthesis (Rodén, 1980, In: The biochemistry of glycoproteins and proteoglycans, Lennarz, W. J., ed, pp. 269-314, Plenum Publishers, New York, Lond*on.).* XT activity was found to be present in the cisternae of the rough endoplasmatic reticulum of various species (Hoffmann et al., 1984, Connect. Tissue. Res. 12, 151-164), and it was shown that the enzyme is secreted from the endoplasmatic reticulum into the extracellular space together with chondroitin sulfate proteoglycans (Kähnert et al., 1991, Eur. J. Clin. Chem. Clin. Biochem. 29, 624-625*;* Götting et al., 1999, J. Invest. Dermatol. 112, 919-924*).* However, the processes resulting in the release of XT from the endoplasmatic reticulum or the Golgi compartments and the role of XT in the extracellular matrix are not yet known.

As XT is the initial step enzyme in the biosynthesis of the glycosaminoglycan linkage region and as it is secreted into the extracellular matrix to a great extent, XT activity was proposed to be a diagnostic marker for the determination of an enhanced proteoglycan biosynthesis and of tissue destruction (Weilke et al., 1997, Clin. Chem. 43, 45-51). XT activities in the synovial fluid were found to be significantly increased in chronic inflammatory joint diseases (Kleesiek et al., 1987, J. Clin. Chem. Clin. Biochem. 25, 473-481). Recent studies have shown that serum XT activity is a biochemical marker for the determination of fibrotic activity in systemic sclerosis (Götting et al.,1999, *l.c.;* Götting et al., 2000, Acta Derm. Venereol. 80, 60-61.).

Up to now there was no success to isolate or characterize xylosyltransferase (XT), however some methods were described to measure and determine the activity of said enzyme from blood or body fluid samples of patients showing pathological effects such as scleroderma or chronic joint diseases (Stoolmiller, 1972, J. Biol. Chem. 247, 3525-3532*).* The samples were incubated with UDP-[¹⁴C]xylose and an appropiate acceptor. The incorporated radioactivity indicated the amount of XT activity. Acceptors used so far are proteoglycans, silk fibroin (Campbell et al., 1984, Anal. Biochem. 137, 505-516*)* and several peptides (Bourdon et al., 1987, Proc. Natl. Acad. Sci. USA 84, 3194-3198*).* However, the all activity tests used herein did not allow precise determination of the lower XT activity in serum. Goetting et al. (J. Prot. Chem. 17, 1998, 295-303) describe the partial purification of XT enzyme from the culture supernatant of JAR choriocarcinoma cells using a sensitive assay involving the more specific acceptor recombinant protein bikunin, which is the inhibitory component of human inter-α-trypsin inhibitor. Bikunin carries a single chondroitin, which is essential for the structure of the inhibitor. The chondroitin sulfate attachement site in the N-terminal region contains all elements responsible for recognition by XT. The complete recognition sequence is composed of the amino acids a-a-a-a-G-S-G-a-b-a, with a = E or D and b = G, E or D. This sequence was confirmed by determination of the Michaelis-Menten (Kₘ) constants for *in vitro* xylosylation of different synthetic proteins and peptides using an enriched XT preparation from conditioned cell culture

supernatant of human chondrocytes. The constant was determined to be 22 µM, which was decreased 9-fold in comparison to deglycosylated core protein from bovine cartilage (188 µM) (Brinkmann et al., 1997, J. Biol. Chem. 272, 11171-11175*).* With recombinant bikunin as acceptor, a sensitive assay was developed that allows precise determination of XT activity in human serum and other body fluids *(see:* Weilke et al., *1997, l.c.).* Using this assay an increased xylosyltransferase activity was determined in blood of patients with sclerotic processes of scleroderma, closely related to an elevated proteoglycan biosynthesis (Götting et al., 1999, l.c.).

The biosynthesis of glycosaminoglycans requires the coordinated action of a large number of glycosyltransferases. Isolation and cloning of these glycosyltransferases has been targeted for a long time, since the majority of these enzymes are only present in minute amounts. The structure and sequence of the glycosyltransferases involved in biosynthesis of the common glycosaminoglycan-protein linkage region has long remained unknown. Recent cDNA cloning of galactosyltransferase I (Okajima et al., 1999, J. Biol. Chem. 274, 22915-22918) and glucuruonyltransferase I (Kitagawa et al., 1998, J. Biol. Chem. 273, 6615-6618) identified 2 of the at least 4 enzymes involved in synthesis of the GlcAp1-3Galβ1-3Galβ1-4Xylβ1-O-Ser structure.

Isolation, purification and characterization of XT involved in biosynthesis of the common carbohydrate-protein linkage structure has been hampered by difficulties in obtaining a sufficient amount of the source material. Since, as above mentioned, XT can be used as an additional biochemical marker for the determination of sclerotic activity in systemic sclerosis and some inflammatory disorders, it is a real need for an isolated, highly purified XT which can be produced by recombinant methods, which is necessary for diagnostic and therapeutic purposes. Moreover, the knowledge of the cDNA sequence of XT allows to use it on gene level such as in gene diagnostic or gene therapy.

### Definitions

- Above and below the term "XT" means xylosyltransferase (UDP-D-xylose:proteoglycan core protein β-D-xylosyltransferase (EC 2.4.2.26) deriving from any origin and includes all possible isoforms, if not otherwise pointed out. The term "hXT" has the meaning of human XT; the term "rXT" of rat XT. The terms "XT-I" , "XT-II", "hXT-I", "hXT-II", "rXT-I2, "rXT-II" mean the specific isoforms 1 and 2 of XT according to the invention, wherein h and r have the indicated meanings.
- Above and below the term "protein" means a protein, a protein fragment or a peptide, if not otherwise explained.
- Above and below the term "recombinant protein" is defined as a protein which was produced by recombinant and biotechnological methods.
- Above and below the term "hXT" or "XT protein(s)" has the meaning of (a) protein(s) deriving from any source, if not otherwise stated out, having the biological activity and / or function *of UDP-D-xylose:proteoglycan core protein* β*-D-xylosyltransferase.*
- Above and below the term "a protein having the biological activity of UDP-D-xylose:proteoglycan core protein β-D-xylosyltransferase " is defined as a protein which has XT - identical or XT - like functions and activities and comprises XT itself and possible mutations, variants, isoforms thereof, including insertions, deletions and substitutions of one ore more amino acids. The term includes also fragments or longer forms of XT as well as dimeric or multimeric forms thereof showing XT functions.
- Above and below the term "isoform" means a second naturally occurring enzyme having the same biological activity as a first naturally occurring enzyme, however differing by another amino acid sequence.

### Summary of the Invention

It was found that XT can be purified 4,700-fold with 1% yield from serum-free JAR choriocarcinoma cell culture supernatant. The isolation procedure includes a combination of ammonium sulfate precipitation, heparin affinity chromatography, ion exchange chromatography and protamine affinity chromatography. Amongst other proteins an unknown protein was detected by matrix-assisted laser desorption ionization mass spectrometry-time of flight analysis (MALDI-TOF) in the purified sample. The molecular weight of this isolated and purified XT protein was determined as 120.000 by SDS-polyacrylamide gel electrophoresis. The isolated protein was enzymatically cleaved by trypsin and endoproteinase Lys-C. Peptide fragments were sequenced by Edman degradation. Searches with the amino acid sequences in protein and EST databases showed no homology to known sequences. XT was enriched by immunoaffinity chromatography with an immobilized antibody against a synthetic peptide deduced from the sequenced peptide fragments and was specifically eluted with the antigen. In addition, XT was purified alternatively with an aprotinin affinity chromatography and was detected by western blot analysis in the enzyme-containing fraction.

Based on the partial amino acid sequence of their isolated and purified new enzyme (XT) derived from human JAR choriocarcinoma cell culture supernatant a novel cDNA was isolated according to the invention, encoding human XT-I enzyme using the degenerate reverse transcriptase-polymerase chain reaction method. The enzyme belongs to a novel family of glycosyltransferases having no homology to proteins of prior art. 5'- and 3'-RACE were performed to isolate a novel cDNA fragment of 3726 bp with a single open reading frame encoding at least 827 amino acids with a molecular mass of 91.000. The human XT-1 gene was located on chromosome 16p13.1 using radiation hybrid mapping, and extracts from CHO-K1 cells transfected with the XT-I cDNA in an expression vector exhibited marked XT activity. Furthermore, a new 3608-bp cDNA fragment encoding a novel protein of 865 amino acids was also isolated by PCR using degenerate primers based on the amino acid sequence of human XT-I. The amino acid sequence of this XT-II isoform displays 55% identity to the human XT-I. The XT-II gene is located on chromosome 17q21.3-17q22, and the exon/intron structure of the 15 kb gene was determined. RT-PCR analyses of XT-I and XT-II mRNA from various tissues confirmed that both XT-I and XT-II transcripts are ubiquitously expressed in the human tissues, although with different levels of transcription. Furthermore, the cDNAs encoding XT-I and XT-II from rat were cloned. The deduced amino acid sequences of rat xylosyltransferases displayed 94% identity to the corresponding human enzyme.

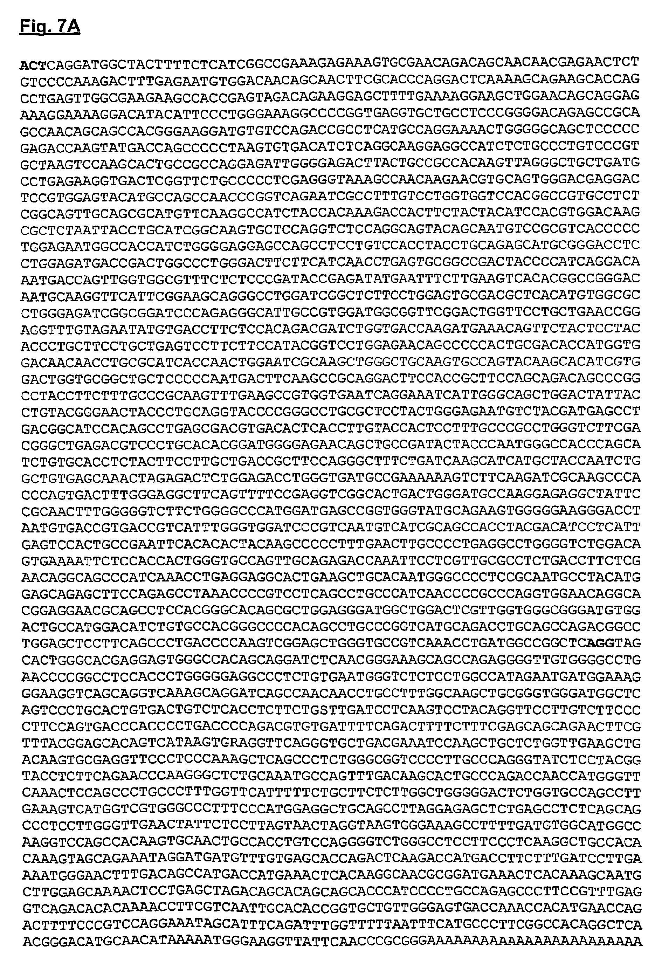

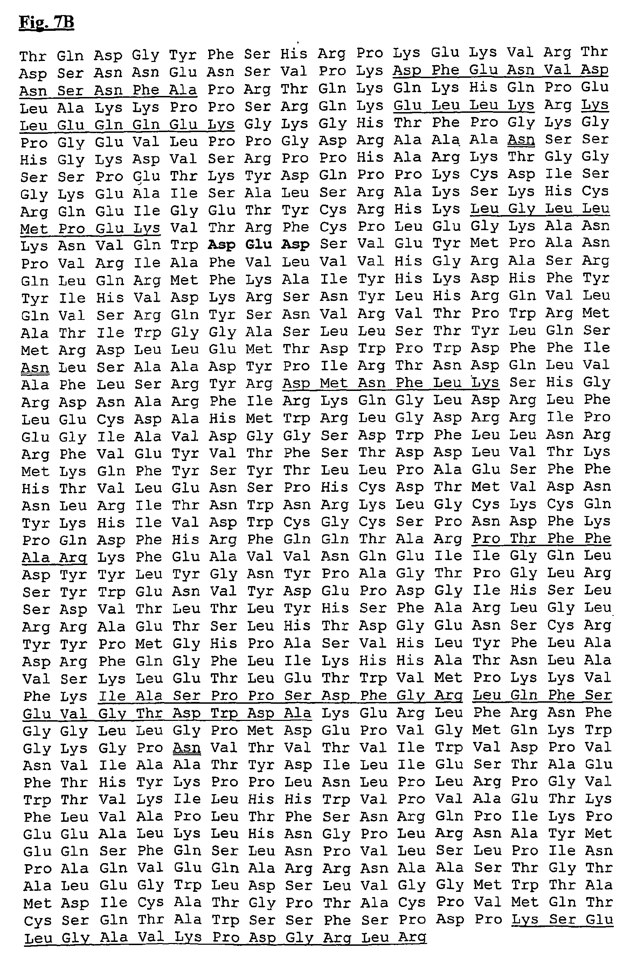

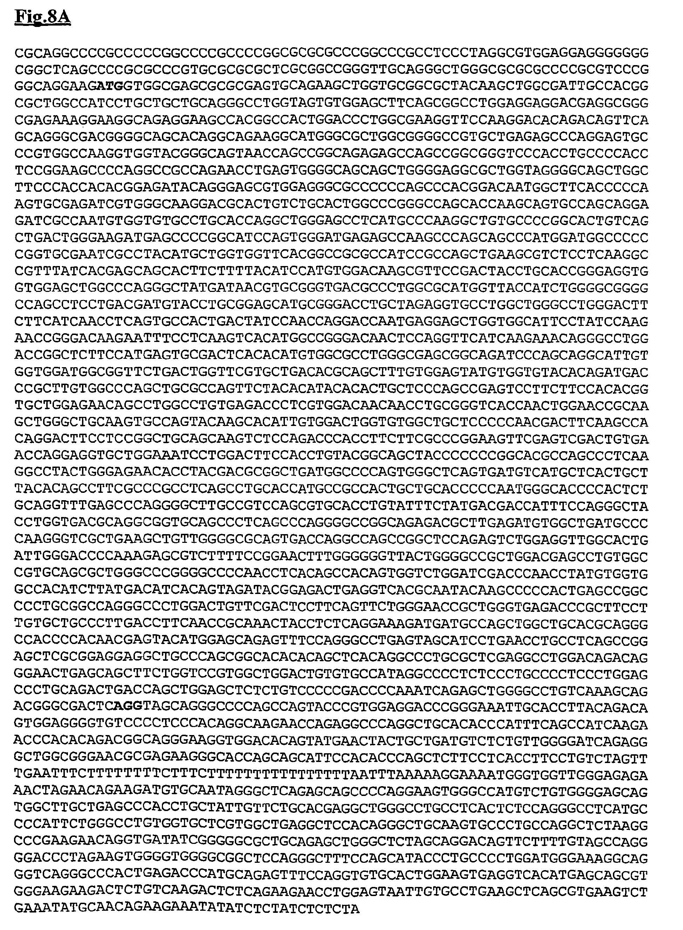

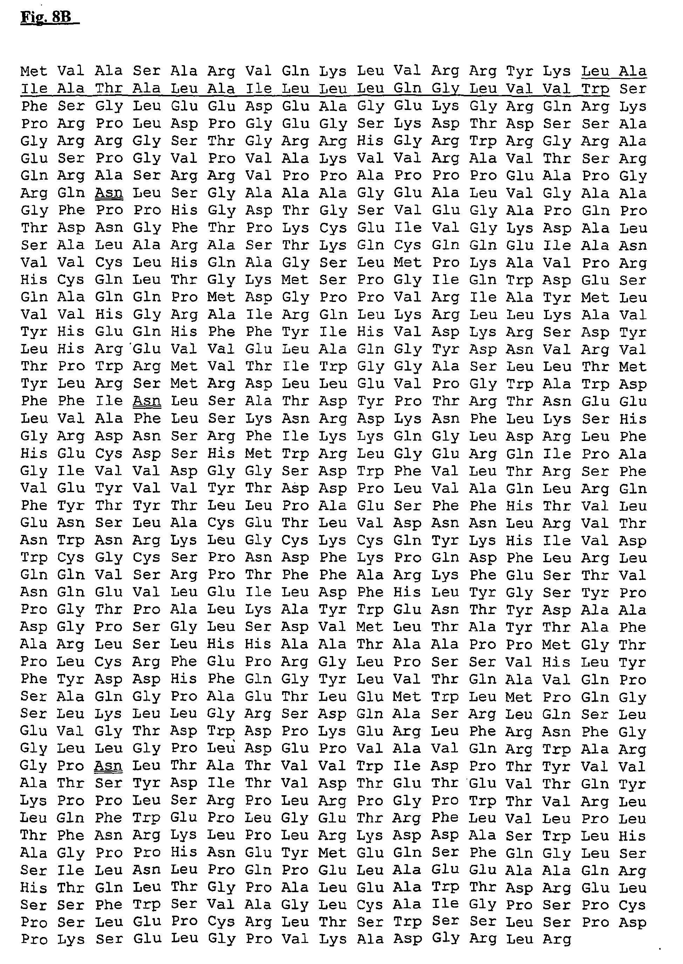

Therefore, it is an object of this invention to provide the following subject-matters:
- An isoform of *UDP-D-xylose:proteoglycan core protein* β*-D-xylosyltransferase*(XT);
- a protein comprising a sequence of said isoform or a fragment thereof, having the biological activity of XT;
- a corresponding protein deriving from human or rat sources (hXT, rXT);
- a corresponding protein isolated from specific human tissue, wherein said hXT has a molecular weight of 120.000 under SDS PAGE conditions;
- a corresponding recombinant protein, wherein said protein is hXT-I comprising at least 827 amino acids and having the amino acid sequence as depicted in Fig. 7B;
- a corresponding isoform of hXT-I, termed as hXT-II, comprising 865 amino acids, exhibiting approximately 55% overall sequence identity to human hXT-1, and having, in more detail, the amino acid sequence as depicted in Fig. 8B.
- a process for manufacturing recombinant two isoforms of hXT (hXT-1 / hXT-II) comprising the following steps:
   (i) culturing human tissue cells, preferably JAR choriocarcinoma cells (ATCC HTB-144), having an enhanced activity of hXT (UDP-D-xylose:proteoglycan core protein β-D-xylosyltransferase [EC 2.4.2.26]) by adapting the cells to hollow fiber culture conditions in a serum-free medium,
   (ii) purifying and isolating hXT with a molecular weight of 120.000 (SDS-PAGE) from the cell supernatant obtained according to step (i) by heparin affinity chromatography and protamine chloride or aprotinin affinity chromatography,
   (iii) enzymatically digesting purified hXT of step (ii) and designing primers from the separated proteolytic fragments,
   (iv) PCR-based cloning of hXT-1 or hXT-II cDNA using proteolytic fragments of step (iii), and
   (v) expressing recombinant hXT-1 or hXT-II protein using an expression vector comprising the DNA sequence of hXT-I or hXT-II.
- recombinant forms of hXT and corresponding DNA sequences as specified in Figures 7 and 8.
- pharmaceutical compositions comprising a XT protein as defined above and below, optionally together with a suitable pharmacologically acceptable carrier, diluent or excipient;
- uses of said XT proteins for the manufacture of a medicament for the treatment of XT relevant diseases and disorders, wherein the xylosyltransferase enzymes according to the invention can either be used directly as therapeutic drug in pathological situations where a deficiency of said enzyme and its isoforms can be detected;
- uses of said XT enzymes for the manufacture of a medicament for the treatment of diseases and disorders which are caused or accompanied by increased levels of said enzymes, e.g. in sclerotic diseases and chronic joint diseases, wherein said medicament is an inhibitor or antagonist of said XT proteins;
- uses of said XT proteins as diagnostic markers for the diagnosis of above- and below-mentioned diseases and pathological symptoms and uses of said DNA molecules as gene markers;

### Detailed Description:

### General

*UDP-D-xylose: proteoglycan core protein* β*-D-xylosyltransferase* can be isolated and purified from JAR choriocarcinoma cell culture. The isolated protein according to the invention is a single stranded polypeptide with a molecular weight of 120.000. The protein was enzymatically cleaved and eleven peptide fragments were sequenced by Edman degradation (see Examples). XT is present only in very small amounts in animal tissues but unlike other glycosyltransferases, more than 90% of XT is enriched in the medium of cultured cells. The highest secretion of XT activity was measured in JAR choriocarcinoma cell culture, in which sternal cartilage chondrocytes and 21 different human cell lines were examined. To produce a highly enriched XT solution for the isolation of XT, JAR choriocarcinoma cells were adapted to hollow fiber culture conditions using a novel bioreactor (TECNOMOUSE) and Ultradoma-PF medium without serum addition as nutrient. For purification of XT a combination of classic separation methods and new affinity matrices was employed. Therefore, a heparin matrix was used as an affinity ligand for the XT. When applied to immobilized heparin, XT was completely adsorbed at the matrix and the XT activity was eluted only with a high salt concentration after most contaminating proteins have been removed from the matrix. Protamine chloride is well-known as cationic activator for several sulfotransferases, so the effect of protamine chloride on the XT was investigated. An increased XT activity was measured when protamine chloride was added to the XT assay solution (see Examples), indicating an interaction of these arginine rich proteins with XT. Therefore, a protamine chloride affinity matrix was synthesized using an aldehyde activated perfusion medium as support. The interaction of XT with this affinity matrix resulted in a 13.6 times enrichment of XT. The protamine chloride affinity chromatography was the most efficient purification step during the isolation of the XT. Immobilized aprotinin, a Kunitz-type proteinase inhibitor, was found to be another appropriate affinity matrix for enrichment of XT, as it was able to adsorb XT quantitatively. Therefore, it was used for alternative purification of XT. Different lines of evidence showed that the isolated protein corresponds to the XT: (a) XT activity was enriched using immobilized antibodies raised against a synthetic peptide deduced from the 120.000 protein, and the XT activity could be competitively eluted with this peptide. (b) Immunoblot analysis of aprotinin affinity purified XT corresponds with the 120.000 protein. However, non-reducing and non-denaturating gel filtration chromatography with heparin affinity-purified XT from JAR cell culture supernatant shows an additional peak of XT activity at a molecular weight of approximately 500.000. XT is associated with proteoglycans. Treatment of XT with N-glycosidase F resulted in a decrease of the molecular weight from 120.000 to 116.000, suggesting that the XT is a glycoprotein. A comparison of the molecular mass of XT with other glycosyltransferases involved in biosynthesis of proteoglycans shows that the XT is larger than the other enzymes. Another difference is that nearly all proteoglycan glycosyltransferases are tightly bound to the membrane of the endoplasmic reticulum, whereas XT is secreted into the extracellular space. XT according to the invention contains like many glycosyltransferases a DxD motif, suggesting that this motif is involved in binding the metal-ion cofactor and the donator substrate (Gastinel et al., 1999, EMBO J. 18, 3546-3557). A DxD sequence was also found in peptide 8 obtained from the enzymatically cleaved XT.

The invention discloses for the first time the molecular cloning and expression of human and rat XT. Surprisingly, XT is found in at least two isomeric forms, which are termed according to the invention as XT-I and XT-II. Based on the amino acid sequence a novel cDNA was cloned, which encodes a protein of at least 827 amino acids with a molecular mass of 91.000. A DxD motif was identified in the XT-I amino acid sequence using hydrophobic cluster analysis. This motif has been observed in many glycosyltransferases, and is involved in the coordination of a divalent cation in the binding of the nucleotide-sugar (Breton & Imberty, 1999, Curr. Opin. Struct. Biol. 9, 563-571.). The translation initiation codon of the XT-I cDNA could not be yet cloned probably due to strong secondary structures in the 5'-region of the XT-I mRNA. The XT-I protein isolated from human JAR choriocarcinoma cell culture supernatant migrated on SDS-PAGE with a molecular mass of 120.000 and the protein size could be further reduced after N-glycanase digestion. These findings indicate that the cloned cDNA represents at least 90% of the coding region of human XT-I.

Another cDNA was completely identified from human placenta RNA, which was similar but not identical to the hXT-I cDNA. The new cDNA encodes a protein of 865 amino acids with a molecular weight of 97.000. This novel protein termed XT-II has a proline-rich region located near the amino-terminus and the type II transmembrane topology characteristic of many other glycosyltransferases cloned to date. The hXT-II protein was similar to the human XT-I with an overall sequence identity of 55%. The similarity of the predicted amino acid sequences was low (<10%) at the amino-terminal end. However, large stretches at the C-terminal region, where the catalytic domain was found to be located in glycosyltransferases, are very conserved with an identity of more than 80% in both proteins. These findings let conclude that the XT-II gene encodes another human xylosyltransferase, although the catalytic activity and the biological role of XT-II remain to be elucidated in detail.

Since alterations in XT activity have been reported to be associated with fibrotic and sclerotic alterations of connective tissue (Götting *et al*., 1999; Götting *et al*., 2000, l.c.), the present findings provide molecular tools to study the function and the regulated expression of human XT as well as the molecular mechanisms of these diseases.

### Production of XT

JAR choriocarcinoma cells which had been adapted to growth in the serum-free Ultradoma-PF medium secreted XT activity into the cell culture supernatant. During the exponential growth XT activity and total protein concentration in the supernatant of a traditional cell culture system (T-flasks) were determined as 0.2 mU/l and 0.1 g/l, respectively. The cells were cultivated using three "Tecnomouse"® bioreactor systems. Each bioreactor contained five culture casettes. About 10⁷ cells per culture casette were inoculated and medium probes of about 0.5 ml were taken every day to determine the viability of the cells and the glucose, and lactate concentration as well as the XT activity of the cell culture supernatant. The cells amounted in the probes varies from 10⁵ to 10⁷ cells per ml with viability between 40 and 70%. XT production increased within three weeks after cell inoculation, reaching a plateau of approximately 30 mU/l. After three months the culture cassette was removed. Harvesting was carried out every two days, collecting 10 ml cell culture supernatant per culture cassette. The cell-free supernatant was collected and stored at -75°C. Mean XT activity in the supernatant of the high density culture was determined as 29.0 mU/l, while the total protein concentration was estimated as 4.8 g/l. In total 18.5 1 high density cell culture supernatant was collected and yielded 535.8 mU XT.

### Isolation and purification of XT from cell culture supernatant

XT was purified form 18.5 l supernatant (equivalent to 2.000 l normal cell culture supernatant) of serum-free cultivated JAR choriocarcinoma cells to an apparent homogeneity of about 4700-fold purification. A summary of the purification steps for isolation of XT is shown in Table 1. XT activity of the crude supernatant was approximately 0.006 mU/mg protein. The purification method according to the invention comprises four, preferably five different steps. It is possible, that one step can be achieved more than once if necessary.

Step 1: Fractionated ammonium sulfate precipitation XT of the ammonium sulfate precipitable fraction was dissolved in 0.461 buffer A with solubilization of 79.5% of the original activity.

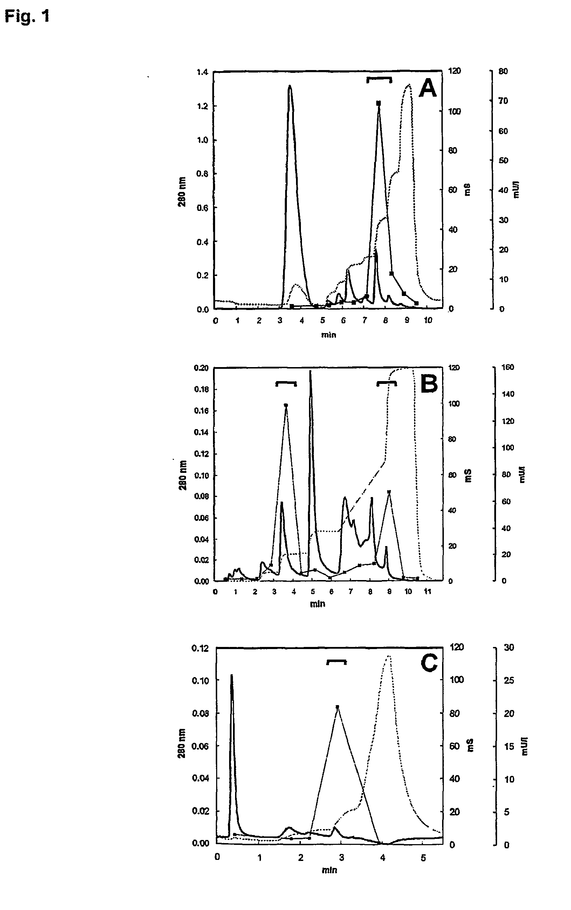

Step 2: Heparin affinity chromatography on POROS 20 HE 4 ml of XT-enriched solution from step 1 was loaded onto the POROS 20 HE column. XT activity was completely retained on the column. More than 70% of total protein passed through the column. Contaminating protein was eluted at a low NaCl concentration. 44% of the XT activity bound to the heparin matrix emerged at 0.5 M NaCl (Fig. 1A).

Step 3: Ion exchange chromatography on POROS 20 HQ 4 ml of the desalted XT-containing fraction from step 2 was loaded onto the POROS 20 HQ column equilibrated in buffer A. More than 98% of the XT activity bound to the resin. The column was then eluted stepwise with NaCl in buffer A (Fig. 1B). XT-containing fractions were collected.

Step 4: Affinity chromatography on protamine chloride The product of step 3 was desalted and concentrated using dia- and ultrafiltration. 100 µl of the protein solution was applied to the POROS protamine chloride column previously equilibrated with buffer A. Approximately 95% of the transferase activity bound to the column, whereas 75% of the contaminating protein did not. Additional proteins were eluted with buffer A containing low NaCl concentrations. Enzyme activity was eluted at approximately 0.15 M NaCl (Fig. 1C). The enzyme activity was stable for at least 6 months at -75°C.

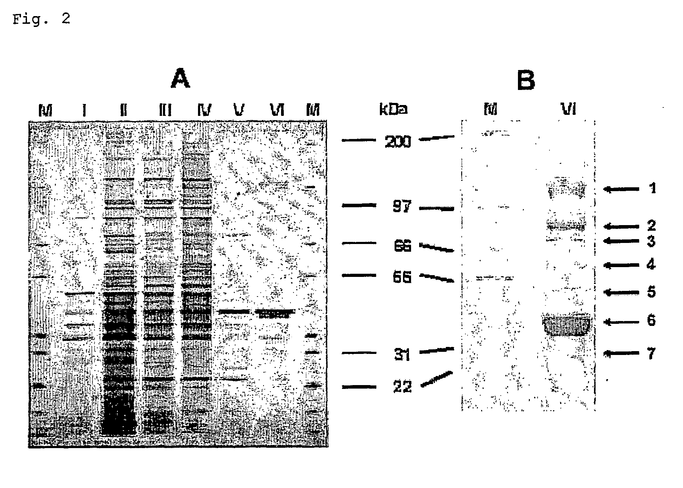

Step 5: SDS-Polyacrylamide gel electrophoresis (SDS-PAGE) XT-containing fractions from step 1-4 were subjected to SDS-PAGE on a 4-12% gradient polyacrylamide gel (Fig. 2, panel A). Coomassie-stained protein bands were excised and characterized by MALDI-TOF mass spectrometry after tryptic digestion. The molecular weight of an unknown protein was determined as 120.000 (Fig. 2, panel B).

Step 5 is an optionally step.

**Table 1:**

| Summary of single purification steps employed for isolation of XT from 18.5 1 of high density JAR cell culture supernatant. | | | | | | |
|---|---|---|---|---|---|---|
| Step | Volume | Total activity | Total protein | Spec. activity | Purification | Recovery |
| | ml | 10-3 x units | mg | 10-3 x units/mg | -fold | % |
| JAR high density cell culture Supernatant | 18,500 | 535.8 | 89,355.0 | 0.006 | 1 | 100 |
| Ammonium sulfate precipitation | 460 | 426.0 | 8,937.8 | 0.048 | 8 | 79 |
| Heparin affinity chromatography | 50 | 108.3 | 473.0 | 0.229 | 40 | 35 |
| Ion exchange Chromatography | 5 | 91.0 | 43.1 | 2.090 | 348 | 17 |
| Protamine affinity chromatography | 1 | 6.83 | 0.24 | 28.458 | 4,743 | 1 |

*Amino acid sequence analysis of XT.* The MW 120.000 protein from the excised band was digested with trypsin and endoproteinase Lys-C. The proteolytic fragments were separated by reversed-phase HPLC, and selected peptides were subjected to automatic amino acid sequence analysis. Table II shows the obtained 11 amino acid sequences determined by Edman degradation and mass spectrometry.

**Table II. X represent a not identified residue. The masses of three peptides were observed in the MALDI mass spectrum of the enzymatically digested MW 120.000 protein. Calculated mass values were obtained from the sequence obtained.**

| **Peptide** | **observed mass** | **calculated mass** |
|---|---|---|
| | (M + H⁺) | (M + H⁺) |
| (1)ELGAK | | |
| (2)ELLK | | |
| (3)DMNFLK | | |
| (4)IASPPSDFGR | 1045.5 | 1045.5 |
| (5)LLLD | | |
| (6)DFENVDNSNFAPR | 1524.7 | 1525.7 |
| (7)PTFFAR | | |
| (8)LQFSEVGTDXDA | | |
| (9)ELGAVKPDGRL | 1152.6 | 1153.7 |
| (10)ELLKRKLEQQEK | | |
| (11)LGLLMPEK | | |

### Immunochemical detection of XT

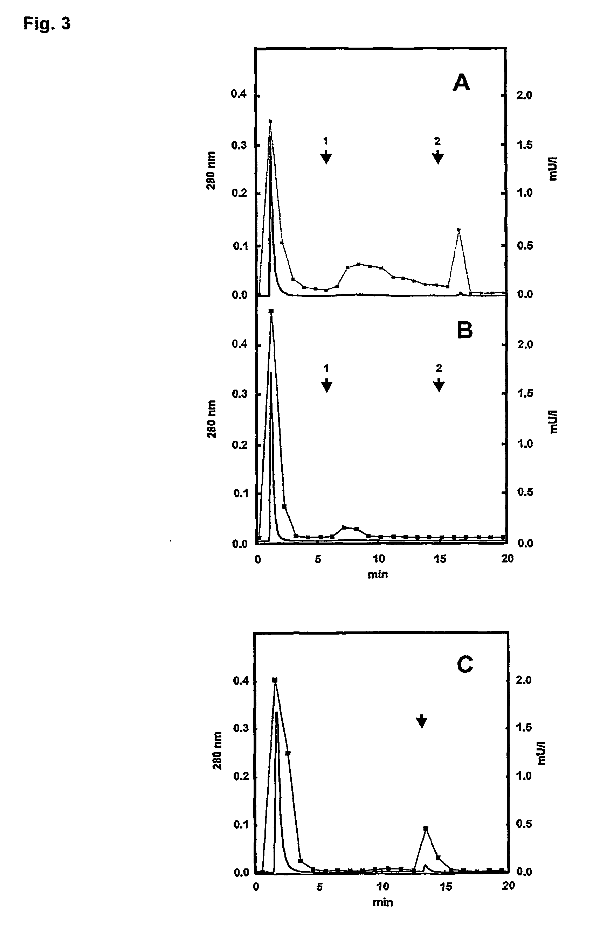

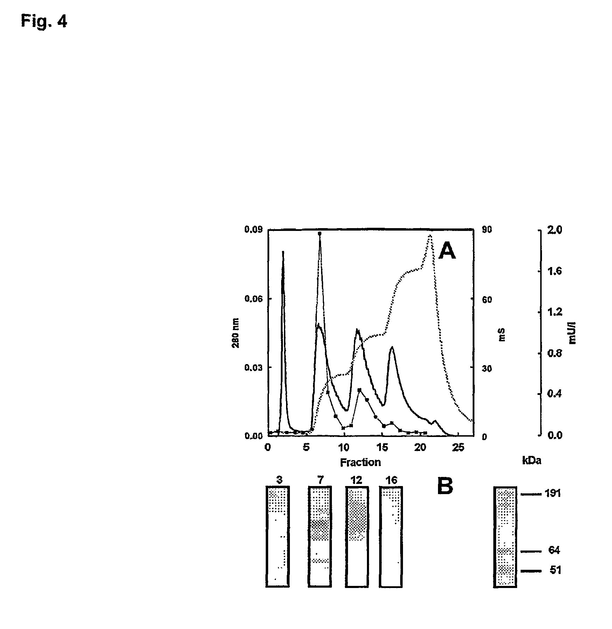

Polyclonal antibodies against the synthetic peptide CSRQKELLKRKLEQQEK deduced from the peptides 2 and 10 of the enzymatically cleaved MW 120.000 protein were covalently bound on POROS 20 PA. About 50% of the XT activity of an applied sample was bound (Fig. 3, panel A) when a partially purified XT sample obtained by heparin affinity chromatography (purification step 2) was loaded onto the column. 58% of the adsorbed XT activity was eluted with 150 mM NaCl, and the rest was eluted with 12 mM HCl. Furthermore, the adsorbed XT activity was also eluted from the solid phase when 100 µl (1 mg/ml) of the synthetic peptide was added to the mobile phase (Fig. 3, panel C). When immobilized preimmune serum was used as affinity matrix (negative control) no XT activity was adsorbed to or eluted from the matrix (Fig. 3, panel B). The desalted XT fraction after heparin affinity chromatography (purification step 2) was loaded on an aprotinin affinity column. The elution profile shows four major protein peaks (Fig. 4, panel I). 61% of the XT activity adsorbed to the aprotinin matrix emerged at 0.30 M NaCl and another 21 % at 0.54 M NaCl. A single MW 120.000 band of the XT-containing fractions was detected by western blot analysis with the polyclonal antibodies (Fig. 4, panel B).

### Determination of the molecular weight of XT

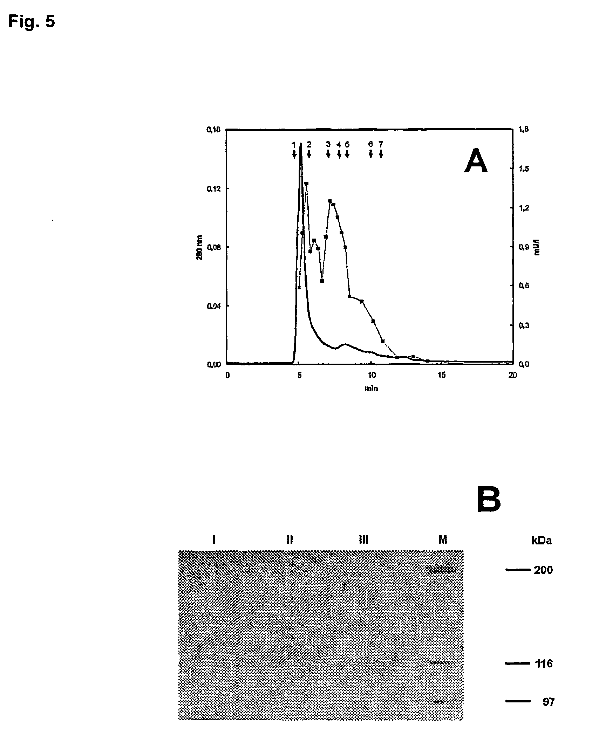

100 µl of heparin affinity purified XT was separated under non-reducing and non-denaturating conditions using a TSK G3000 SW column. Two XT activity maxima were detected at MW 500.000 and 120.000 (Fig. 5, panel A). The molecular mass of the MW 120.000 protein was reduced about 3 % after N-glycosidase F digestion as shown by SDS-PAGE (Fig. 5, panel B).

### PCR-based cloning of human XT-I cDNA

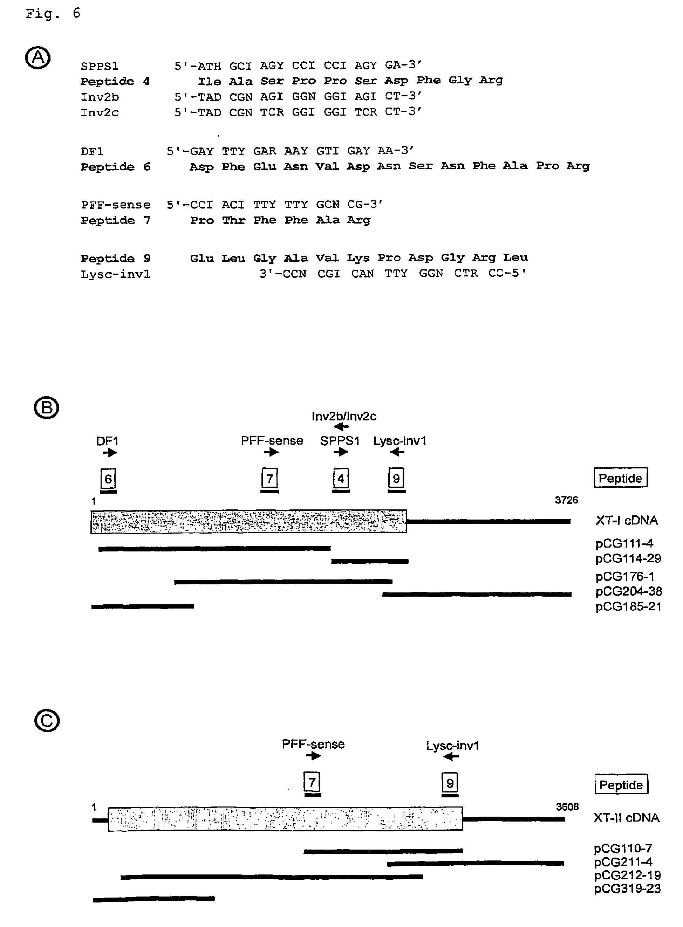

Based upon the amino acid sequence of 4 peptides degenerate primers were designed for cloning the XT-I cDNA (Figure 6). When primers SPPS1 and Lysc-inv were used in a PCR with the first strand cDNA of SW1353 chondrosarcoma poly(A)⁺ mRNA as template, a major band of approximately 690 bp was observed. After subcloning and sequencing a previously unknown DNA sequence was obtained from clone pCG114-29. PCR amplification with the primers DF1 and a mixture of Inv2b and Inv2c resulted in a 1724 bp fragment, which was subcloned and sequenced. The deduced amino acid sequence of the clone pCG111-4 was identical to 6 of the sequenced peptides from human XT-I. The cloning strategy of rapid amplification of cDNA ends (RACE) (Chenchik et al., 1996, BioTechniques 21, 526-534) was employed to clone the complete coding sequence of the XT-I cDNA. The largest DNA fragment obtained from the 3'-RACE reaction was 1.6 kb and consisted of a 3'-untranslated region of 1240 bp. The 5' RACE reaction was performed with different primers derived from the nucleotide sequence of XT-I cDNA, but the 5'-untranslated region of human XT-I cDNA could not be cloned using this method. All DNA fragments obtained contained just the known cDNA sequence and an additional 80 bp of coding sequence. Thus, a PCR-based screening approach using cDNA libraries as template was employed for cloning of the 5'-untranslated region. All DNA fragments obtained from the screening of 3 different human cDNA libraries stopped at the same nucleotide, indicating that stable secondary structures of the XT-I mRNA prevent the synthesis of cDNA of the 5' untranslated region during the reverse transcription reaction. However, the translation initiation codon of human XT-I could not be yet cloned. The combined cDNA of human XT-I contained 3726 bp (Fig. 7A) with a single open reading frame encoding at least 827 amino acids with a molecular mass of a least 91.000. The deduced amino acid sequence contained 3 potential N-glycosylation sites (Figure 7B). Analysis of the amino acid sequence using the hydrophobic cluster analysis (Gaboriaud *et al*., 1987, ) revealed the presence of a common DxD motif at position 182, which has been shown to be essential for binding nucleotide-sugars in glycosyltransferases.

### PCR-based cloning of human XT-II isoform cDNA

The degenerate primers PFF-sense and Lysc-inv1 which were designed upon the amino acid sequence of proteolytic cleaved peptides of human XT-I were used in a PCR reaction with first strand cDNA of placenta poly(A)⁺ mRNA as template. The PCR amplification resulted in a minor band of 1.1 kb, which was cloned and sequenced. The determined nucleotide sequence was similar but not identical to the XT-I cDNA sequence, indicating that the fragment encodes a XT-II isoform. To clone the complete coding sequence of the novel cDNA, the RACE strategy was employed. The cDNA finally obtained was 3608 bp (Figure 8A) and contained a single open reading frame encoding a protein of 865 amino acids with a molecular mass of 97.000. The 3'-untranslated region is 850 bp and a 5'-untranslated region of 149 bp was identified with an in-frame stop codon upstream of the ATG codon. A Kyte-Doolittle hydropathy analysis (Kyte & Doolittle, 1982, J. Mol. Biol. 157, 105-132) of the deduced amino acid sequence revealed one potential membrane-spanning region consisting of 16 hydrophobic amino acid residues at position 16 to 32, which appears to result in a type II transmembrane orientation characteristic of many of the glycosyltransferases. The predicted amino acid sequence contains a proline-rich profile pattern from position 110 to 118 and 3 potential N-glycosylation sites (Figure 8B). The human XT-II isoform protein exhibits 55% overall sequence identity to the human XT-I including stretches in the XT-II protein with more than 80% homology to the human
XT-I.

### Identification of the chromosomal localization of the human XT-I and XT-II genes

The gene coding for human XT-I could be assigned to the short arm of chromosome 16 at the region 16p13.1. The radiation hybrid mapping using the Genebridge 4 radiation hybrid screening panel located the position of the PCR fragment 5.98 cR distal to the STS marker CHLC.GATA42E11 and 4.40 cR proximal to marker D16S499. The XT-II gene was identified on chromosome 17 at the position 17q21.3-17q22 and was found to be located distal to the STS marker WI-11424 and proximal to the marker WI-14315.

### Expression of recombinant XT

To prove the function of the cDNA product, a recombinant form of XT-I was generated by fusing the cloned XT-I to an aminoterminal peptide tag. The fused protein was expressed in CHO-K1 cells and absorbed from the medium by immunoprecipitation with anti-*Xpress-*antibodies and protein G agarose beads to eliminate endogenous XT activity. The enzyme-bound beads were used as an enzyme source and assayed for XT activity as shown in Table 3 (see below). No detectable XT activity was recovered by the affinity purification from a control transfection sample. The substrate specificity of the recombinant XT-I was similar to that of the XT-I isolated from human body fluids and cell culture supernatants. The recovered enzyme activity of the recombinantly expressed XT-I could be completely inhibited by addition of 250 U of heparin. As specific inhibition of human XT activity by heparin has been demonstrated previously (Kleesiek *et al*., 1987, l.c.), these results clearly indicate that the expressed protein is the human XT. However, no enzymatic transfer of xylose to the acceptor peptides used in this study was observed when expressing XT-II fused to the aminoterminal peptide tag in CHO-K1 cells.

To identify the XT-I reaction products, the bikunin peptide was labeled with [¹⁴C]-D-xylose using the XT-I-bound beads as an enzyme source. The products were subsequently subjected to the linkage-specific digestion of the bound [¹⁴C]-D-xylose with α- and β-xylosidase and alkaline β-elimination. Incubation of the reaction products with β-xylosidase resulted in the release of 74% of the incorporated [¹⁴C]xylose, whereas only less than 4% of the peptide-bound xylose was digested after treatment with α-xylosidase. The alkaline cleavage of the O-glycosidic linkage between the xylose and the β-hydroxyamino acid serine in the presence of borohydride lead to the liberation of more than 97% of the enzymatically transferred [¹⁴C]xylose. In all the experiments performed no significant amount of [¹⁴C]-D-xylose was incorporated without the addition of the bikunin peptide as acceptor. These results clearly indicate that a xylose residue was transferred to the hydroxyamino acid serine of the bikunin peptide through a β-linkage. In conclusion the expressed protein was identified as UDP-D-xylose:proteoglycan core protein β-D-xylosyltransferase (EC 2.4.2.26).

**Table 3:**

| *Xylosyltransferase activity of recombinant XT-I expressed in CHO-K1 cells.* XT activity of the enzyme fractions using different acceptors and the inhibition of enzyme activity by addition of heparin is shown. The synthetic bikunin, L-APP and L-APLP2 homologous peptides have been previously proved to be good acceptors for XT mediated xylosylation (Brinkmann *et al*., 1997; Götting *et al*., 1998). No XT activity was detected in samples from mock-transfected cells after affinity purification. n.d., not detected (detection limit, 20 µU/l). | | |
|---|---|---|
| *Acceptor* | *XT Activitiy* | |
| | *pCG227-XT* | |
| | | *+ heparin* |
| Recombinant bikunin | 2854 | n.d. |
| Bikunin peptide QEEGSGGGGQK | 463 | n.d. |
| L-APLP2 peptide SENEGSGMAEQK | 515 | n.d. |
| L-APP peptide TENEGSGLTNIK | 492 | n.d. |
| Peptide SGG | n.d. | n.d. |
| Chondroitin sulfate A | n.d. | n.d. |
| Chondroitin sulfate C | n.d. | n.d. |

### Tissue-specific expression of XT-I and the XT-II isoform

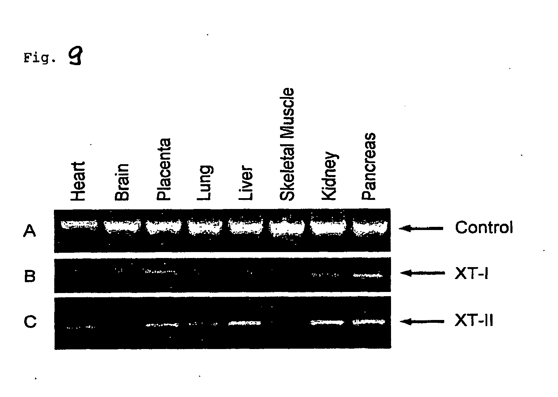

The expression of XT-I and XT-II isoform gene was examined in various human tissues using a RT-PCR based method with normalized first-strand cDNA. Each PCR yielded a single product with predicted nucleotide lengths of 490 bp for XT-I and 717 bp for XT-II, although the amount of the amplified product varied (Figure 9). Amplification of XT-I and XT-II fragments resulted in a product visible by ethidium bromide staining after 36 cycles, whereas the DNA fragment corresponding to the abundant glyceraldehyde-3-phosphate dehydrogenase transcript was visible after 20 cycles. The greatest abundance of XT-I expression was detected in the placenta, kidney and pancreas and only a very weak expression was detected in skeletal muscle. The greatest abundance of XT-II isoform is expressed in the kidney and pancreas.

### Cellular distribution of XT activity in cultured CHO-K1 cells

After an incubation period of 3 days cultured CHO-K1 cells were harvested and the XT activity was determined in the spent culture supernatant and the cell lysates. 92% of the total XT activity was found to be located in the cell culture medium (93.1 mU/10⁶ cells, SD 9.58), whereas only 2% was detected in the cell lysates (2.03 mU/10⁶ cells, SD 0.44). 6% of the total XT activity (5.82 mU/10⁶ cells, SD 2.18) was released from the membrane-containing fractions after addition of the detergence Triton X-100 indicating that less than 1/10th of XT is bound to cellular membranes.

### Pharmaceutical and diagnostic use

The xylosyltransferase enzymes according to the invention can be used directly as therapeutic drug in pathological situations where a deficiency of said enzyme and its isoforms can be detected. As pointed out above XT enzyme may be overexpressed in some diseases and disorders. In these cases inhibitors and / or antagonists of XT-I and or XT-II can be used. Thus, the invention relates also to the use for the manufacture of a medicament for the treatment of diseases which are caused by increased levels of said enzymes, wherein said medicament is an inhibitor or antagonist of xylosyltransferase.

As mentioned above the protein according to this invention can be used as diagnostic means to evaluate pathological conditions. As used herein, the term "pharmaceutically acceptable carrier" means an inert, non toxic solid or liquid filler, diluent or encapsulating material, not reacting adversely with the active compound or with the patient. Suitable, preferably liquid carriers, are well known in the art. The formulations according to the invention may be administered as unit doses containing conventional non-toxic pharmaceutically acceptable carriers, diluents, adjuvants and vehicles which are typical for parenteral administration.

The term "parenteral" includes herein subcutaneous, intravenous, intra-arhcular and intratracheal injection and infusion techniques. Also other administrations such as oral administration and topical application are suitable. Parenteral compositions and combinations are most preferably adminstered intravenously either in a bolus form or as a constant fusion according to known procedures. Tablets and capsules for oral administration contain conventional excipients such as binding agents, fillers, diluents, tableting agents, lubricants, disintegrants, and wetting agents. The tablets may be coated according to methods well known in the art.

Unit doses according to the invention may contain daily required amounts of the protein according to the invention, or sub-multiples thereof to make up the desired dose. The optimum therapeutically acceptable dosage and dose rate for a given patient (mammals, including humans) depends on a variety of factors, such as the activity of the specific active material employed, the age, body weight, general health, sex, diet, time and route of administration, rate of clearance, enzyme activity (units/mg protein), the object of the treatment, i. e., therapy or prophylaxis and the nature of the disease to be treated.

Therefore, in compositions and combinations in a treated patient (in vivo) a pharmaceutical effective daily dose of the protein of this invention (hXT, hXT-I, hXT-II) is between about 0.01 and 100 mg/kg body weight (based on a specific activity of 100 kU/mg), preferably between 0.1 and 10 mg/kg body weight. According to the application form one single dose may contain between 0.5 and 10 mg of hXT.

### Short Description of the Figures

### Figure 1: Purification of XT

*Panel A, Heparin affinity chromatography on POROS 20 HE:* The dissolved ammonium sulfate precipitate from JAR choriocarcinoma supernatant was applied to a POROS 20 HE column. After equilibration with buffer A, the column was eluted stepwise with NaCl ( - ). Protein elution was monitored at wavelength A₂₈₀ ( - ), and fractions of 38 ml were assayed for XT activity (■). The horizontal bracket indicates fractions collected for further purification. *Panel B, Ion exchange chromatography on POROS 20 HQ* : The desalted and concentrated product from heparin affinity chromatography was loaded onto a POROS 20 HQ column. The column was washed with buffer A. Adsorbed proteins were eluted stepwise with NaCl ( - ). Protein elution was monitored at A₂₈₀ ( - ), and fractions of 50 ml were assayed for XT activity (■). The horizontal brackets indicate the fractions collected for further purification. *Panel C, Protamine chloride affinity chromatography:* The desalted and concentrated product from ion exchange chromatography was applied to a protamine chloride POROS column. After a washing step with buffer A, the column was eluted stepwise with NaCl ( - ). Elution was monitored at A₂₈₀ ( - ), and fractions of 6 ml were assayed for XT activity (■). The horizontal bracket indicates the fractions collected.

### Figure 2 SDS-PAGE of XT fractions at various purification steps

*Panel A:* XT fractions at various purification steps were subjected to 4-12% gradient polyacrylamide gel for SDS-PAGE. *Lane I*, JAR cell culture supernatant (crude material); *lane II*, ultrafiltration retentate; *lane III*, dissolved ammonium sulfate precipitate; *lane IV*, protein eluted with NaCl from the POROS 20 HE column; *lane V*, protein eluted with NaCl from the POROS 20 HQ column; *lane VI*, protein eluted with NaCl from the protamine chloride POROS column. *Lane M*, molecular size standard: myosin (200.000), phosphorylase b (97.000), bovine serum albumin (66.000), glutamic dehydrogenase (55.000), carbonic anhydrase (31.000), trypsin inhibitor (22.000). The gel was silver-stained. *Panel B:* Collected XT fractions from protamine affinity chromatography were subjected to SDS-PAGE on a 4-12% gradient polyacrylamide gel. The arrows indicate the stained bands corresponding to the unknown protein, which was shown to be XT (120.000) (*1)*, hexose-6-phosphate dehydrogenase (89.000) *(2*), ezrin (68.600) *(3)*, quiescin Q6 (64.000) *(4)*, plasminogen activator (47.000) *(5)*, aldolase A (39.000) *(6)* and low molecular artefacts *(7)*. All protein bands were excised, enzymatically digested, and the peptide mixture was characterized by MALDI-TOF mass spectrometry. The gel was stained with Coomassie Brilliant Blue.

### Figure 3: Immunoaffinity chromatography of xylosyltransferase

*Panel A,* 100 µl of the desalted XT-containing fractions eluted from heparin affinity matrix was applied to a column of immobilized polyclonal antibodies. After washing with buffer D, the column was eluted as indicated by the *arrow* with buffer D / 0.15 M NaCl (1) followed by 12 mM HCl (2). Fractions of 1 ml were collected into tubes containing 1 ml of 0.1 M Tris/HCl, pH 8.0. Protein elution was monitored at 280 nm (-) and.the XT activities (■) of each fraction were assayed.

*Panel B,* Negative control of the immunoaffinity chromatography with immobilized preimmune serum. For conditions see *Panel A.*

*Panel C*, Immunoaffinity chromatography with immobilized polyclonal antibodies. The column was eluted with buffer D containing 100 µl (1 mg/ml) of the peptide antigen indicated by the arrow. Fractions of 1 ml were collected into tubes containing 1 ml of 0.1 M Tris/HCl, pH 8.0. Protein elution was monitored at 280 nm ( - ) and the XT activities (■) of each fraction were assayed.

### Figure 4: Aprotinin affinity chromatography of partially purified XT and immunoblot arcalysis of the separated fractions

*Panel A,* 200 µl desalted XT fraction from the heparin purification step was applied to an aprotinin column previously equilibrated with buffer A. After washing with buffer A, the adsorbed proteins were eluted stepwise with NaCl ( - ). Protein elution was monitored at A280 (-) and fractions of 2 ml were assayed for XT activity (■).

*Panel B,* Aliquots of fraction 3, fraction 7, fraction 12 and fraction 16 were analyzed by western blot. The MW 120.000 protein was detected in the XT-containing fraction 7 and 12. Immunological detections were performed using a polyclonal rabbit antiserum raised against the synthetic peptide CSRQKELLKRKLEQQEK deduced from the peptides 2 and 10 of the enzymatically cleaved unknown protein. Prestained molecular size standard were myosin (190.000), BSA (64.000), glutamic dehydrogenase (51.000).

### Figure 5 : Gel filtration chromatography and N-glycosidase F digestion

*Panel A,* 100 µl of the XT containing fraction from heparin affinity HPLC was applied to a TSK G3000 SW column (30 cm X 7.5 mm, 10 µm particle size) previously equilibrated with buffer A / 0.15 M NaCl. Elution was performed with the same buffer. Fractions of 200 µl were collected and assayed for XT activity (■). Protein elution was monitored at A₂₈₀ (-). The *arrows* indicate the elution positions of thyroglobulin (669.000) *(*1), ferritin (440.000) *(*2), aldolase (158.000) *(*3), albumin (67.000) *(*4), ovalbumin (43.000) (5), chymotrypsinogen A (25.000) *(*6), and ribonuclease A (13.700) *(*7).

*Panel B,* An aliquot of 1 µg of the MW 120.000 protein was analyzed by SDS-PAGE (I). Aliquots (1 µg) of the MW 120.000 protein were digested with 3,1 x 10 ⁻³ units of N-glycosidase F at 37°C for 1 h (II) and 12 h (III). The samples were then subjected to SDS-PAGE, and protein bands were detected by silver staining. The molecular size standard were myosin (200.000), b-galactosidase (116.000), phosphorylase b (97.000).

### Figure 6: Cloning strategy for human XT-I and XT-II isoform.

(A) The peptides 4, 6, 7, and 9 are sequences that were most favorable for the design of degenerate PCR primers. The amino acid sequence of the peptides was obtained after proteolytic digestion of the purified human XT. The strategy for cloning of XT-I cDNA (B) and XT-II isoform (C) is illustrated. The open reading frame of XT-I and XT-II is shown as a filled box, and the location of the peptides 4, 6, 7 and 9 is illustrated by open boxes. The location and orientation of the degenerate primers employed for cloning of XT-I and XT-II are marked by arrows. XT-I and XT-II cDNA inserts contained within the indicated plasmids were obtained using RT-PCR with degenerate primers (pCG 111-4, pCG 114-29, pCG110-7), 5' RACE (pCG185-21, pCG212-19, pCG319-23, 3' RACE (pCG204-38, pCG211-4) and RT-PCR with gene-specific primers (pCG176-1). H = A + C + T, Y = C + T, R = A + G, N = A +G + C + T, I = deoxyinosine.

### Figure 7A, B: Nucleotide (A) and deduced amino acid (B) sequence of the hXT-I.

The position of the peptide sequences obtained by digestion of the purified human XT are underlined (B). Potential N-glycosylation sites are double underlined. The DxD motif is intensified depicted. The figures are identical with SEQ ID 1 and 2.

### Figure 8A, B: Nucleotide (A) and amino acid (B) sequence of the hXT-II isoform.

Potential N-glycosylation sites are double underlined (B). The putative transmembrane domain is underlined. The figures are identical with SEQ ID 3 and 4.

**Figure 9****:** *Differential expression of the XT-I, XT-II gene in human tissues.* Semiquantitative RT-PCR with normalized first-strand cDNA was used to examine the abundance of XT-I and XT-II transcripts. A 983 bp fragment of the housekeeping gene glyceraldehyde-3-phosphate dehydrogenase cDNA was amplified as control (A). The 490 bp XT-I cDNA fragment (B) and the 717 bp fragment amplified from XT-II isoform cDNA (C) were detected in each tissue indicating that both enzymes are ubiquitously expressed. The arrows indicate the expected position for each PCR product.

The following examples describe the invention in more detail without to limit scope of the technical teaching.

**Example 1:** *Materials for Isolation and Purifications.* Human JAR choriocarcinoma cells were purchased from ATCC (Rockville, MD). Dried UltraDOMA-PF medium was obtained from Bio Whittaker (Vervier, Belgium) and *aqua ad injecta* from Braun (Melsungen, Germany). Heat-inactivated fetal calf serum, Dulbecco's phosphate-buffered saline, antibiotic/antimycotic solution, trypsin-EDTA solution, trypan blue, protamine chloride and the *Bicinchoninic Acid Protein Assay Kit* were purchased from Sigma (Deisenhofen, Germany). Cell culture flasks, serological pipettes, and sterile tubes were purchased from Becton Dickinson (Heidelberg, Germany). The hybrid hollow-fiber bioreactor TECNOMOUSE^{®} was supplied by Integra Biosciences (Fernwald, Germany), the ACA analyzer by Dade Diagnostica (München, Germany) and the Super G analyzer by RLT (Möhnesee, Germany). UDP-[ ¹⁴ C]xylose (9.88 kBq / nmol) came from DuPont (Bad Homburg, Germany), 25 mm diameter nitrocellulose discs from Sartorius (Göttingen, Germany), scintillation mixture and the liquid scintillation counter LS500TD was obtained from Beckman Coulter (Fullerton, CA). Ultrafiltration cells, YM1 membranes and PVDF membranes (Immobilon P) were purchased from Millipore (Eschborn, Germany).

The chromatography media POROS 20 HQ, POROS 20 HE2, POROS 20 AL, POROS 20 EP and the HPLC workstation *Biocad Sprint* were supplied by Perseptive Biosystems (Framingham, MA). The gel filtration column TSK G3000 SW (30 cm x 7.5 mm, 10 µm particle size) was obtained from TosoHaas (Montgomeryville, PA). The MALDI mass spectrometer Reflex II was from Bruker Daltonik GmbH (Bremen, Germany) and protein sequencer Procise 494 cLC was purchased from PE Biosystems (Framingham, MA). Precast polyacrylamide gels, buffers, and NuPAGE electrophoresis system XCell II Mini-Cell and Blot Module were from Novex (San Diego, CA). The synthetic peptide CSRQKELLKRKLEQQEK and the rabbit antiserum were purchased from BioScience (Göttingen, Germany). Peroxidase-conjugated affinipure F(ab')2 fragment goat anti-rabbit IgG (H+L) was purchased from Dianova (Hamburg, Germany). N-glycosidase F was obtained from Roche (Mannheim, Germany).

**Example 2:** *Cell culture.* JAR choriocarcinoma cells releasing XT in the cell culture supernatant were cultured in Ultradoma-PF medium containing 10% heat-inactivated fetal calf serum, 100 U/ml penicillin, 100 µg/ml streptomycin and 250 ng/ml amphotericin B in a humidified atmosphere of 5% CO2 and 95% air at 37°C. After incubation for 24 h in the serum-containing medium, the cell cultivation was adapted to serum-free conditions as described previously (6). Scaling up of XT production was carried out in three hybrid hollow-fiber bioreactors TECNOMOUSE^{®}. During the exponential growth the cells from three 175 cm 2 T-flasks (> 3 x 10⁷ cells) were detached with 0.5% trypsin and 0.2% EDTA in Dulbecco's phosphate-buffered saline by incubation at 37°C for 10 min. After centrifugation (5 min, 1000 x g) of the cell suspension the cell pellet was resuspended in 10 ml 37°C warm serum-free and protein-free Ultradoma-PF medium and washed three times with 20 ml of the same medium. The cell suspension was drawn into a 10 ml syringe and then inoculated into the extracapillary space (EC space) of the reactor. The bioreactor was connected with a 2 liter medium bottle and set to 150 ml/h in the recirculation mode, and the oxygenation pump was set as described in the operating manual. Five days after inoculation a 10 ml syringe was connected to the left hand EC port and 10 ml of cell culture supernatant was harvested from the EC space. The harvesting was continued every two days over a period of 3 months. Glucose and lactate concentration of the cell culture supernatant were controlled using the Super G analyzer and the ACA analyzer, respectively. The 2 liter medium bottle was replaced every three days. The viability of the cells was determined by trypan blue exclusion.

**Example 3:** *Synthesis of the protamine affinity matrix.* Protamine chloride was immobilized as ligand on POROS 20 AL. 30 mg ligand was dissolved in 10 ml of 10 mM phosphate / 0.15 M NaCl, pH 7.4. Alter the protein had been dissolved, 5 ml of 100 mM phosphate / 1.50 M NaCl, pH 7.4 was added. NaCNBH₃ was dissolved in the ligand/buffer solution to a final concentration of 5 mg/ml and 1.0 g POROS 20 AL was suspended in the same solution.

The suspension was mixed gently on a shaker for 1 minute at RT. An additional 2 ml of 100 mM phosphate / 1.50 M NaCl, pH 7.4 was added to the suspension and the mixture was shaken continuously. This step was repeated every five minutes until the mixture volume was 25 ml. After additional shaking for 2 h, the medium was filtered on a sintered glass funnel. The matrix was suspended in 20 ml of 0.2 M Tris/HCl / 5 g/l NaCNBH₃, pH 7.2 and mixed gently on a shaker for 30 min at RT. After the media had been washed in a sintered glass funnel using 100 ml of 10 mM phosphate, pH 7.4, 100 ml of 1.0 M NaCl and another 100 ml of 10 mM phosphate, pH 7.4, the matrix was packed in a PEEK column (4.6 x 50 mm).

**Example 4:** *Synthesis of the aprotinin affinity matrix.* Aprotinin affinity matrix was synthesized according to the synthesis of the protamine affinity matrix, but using aprotinin as ligand. After immobilization of the ligand the matrix was packed in a PEEK column (4.6 x 50 mm).

**Example 5:** *Purification of xylosyltransferase from JAR cell culture supernatant.* Fractionated ammonium sulfate precipitation and chromatography steps were performed at RT, ultrafiltration and diafiltration were carried out at 4°C. 18.5 liters JAR cell culture supernatant collected from three hybrid hollow-fiber bioreactors, TECNOMOUSE^{®}, each containing 5 culture cassettes, was concentrated to 800 ml with ultrafiltration cells using YM1 cellulose membranes. The retentate was centrifuged at 4,000 x g for 1 h. The supernatant was decanted, and the pellet was discarded.

*Steep 1: Fractionated ammonium sulfate precipitation -* Solid ammonium sulfate was added to the supernatant to 28% saturation. After 1 h at RT the suspension was centrifuged at 4,000 x g for 2 h, the supernatant was decanted, and the precipitate was removed. Additional ammonium sulfate was added to the solution to the point of 40% saturation, and the suspension was allowed to stand for 1 h. To recover the precipitate the supernatant was decanted after the suspension was centrifuged at 4,000 x g for 2 h. Before chromatography on immobilized heparin the precipitate was dissolved in 460 ml buffer A (20 mM sodium acetate, pH 6.0).

*Step 2: Heparin affinity chromatography on POROS 20 HE2 -* The step 1 product was passed through a 0.2 µm filter. 4.0 ml of the filtrate was applied to a POROS 20 HE2 column (16 x 100 mm) equilibrated with buffer A at a flow rate of 40 ml/min. After washing the column with 100 ml of buffer A the XT activity was eluted with the same buffer containing NaCl. The NaCl concentration was increased stepwise: 20 ml buffer A / 0.09 M NaCl; 20 ml buffer A / 0.15 M NaCl; 30 ml buffer A / 0.24 M NaCl; 24 ml buffer A / 0.30 M NaCl; 24 ml buffer A / 0.60 M NaCl; 24 ml buffer A / 1.00 M NaCl; and 24 ml buffer A / 1.89 M NaCl. Fractions of 38 ml each were collected and the XT activity was measured. The procedure was repeated 115 times by cyclic chromatography and the fractions containing XT activity (115 x 38 ml) were collected.

*Step 3: Ion exchange chromatography on POROS 20 HQ -* Collected fractions from step 2 were desalted using diafiltration with YM1 cellulose membranes and ultrafiltration cells. After concentration of the desalted protein solution to 0.05 liter using analogous techniques the XT-enriched solution was subjected to ion exchange chromatography. 4.0 ml of the XT solution was applied onto the POROS 20 HQ column (16 x 100 mm) previously equilibrated with buffer A at a flow rate of 40 ml/min. The column was washed with 80 ml buffer A, and the adsorbed protein was eluted stepwise using the same buffer containing 0.07 M NaCl (88 ml), 0.18 M NaCl (120 ml), and 0.36 M NaCl (120 ml) followed by a linear gradient of 0.36 -1.00 M NaCl (200 ml) and another step of buffer A / 2.0 M NaCl (120 ml). 50 ml fractions were collected and assayed for activity and evaluated by SDS-PAGE. Chromatography was repeated 13 times, and the fractions exhibiting XT activity (13 x 50 ml) were collected for affinity chromatography.

*Step 4: Affinity chromatography on protamine chloride-* XT-containing solution from step 3 was desalted as described above and concentrated to 5 ml by ultrafiltration with YM1 cellulose membranes. The ultrafiltration product was passed through a 0.2 µm filter. 100 µ1 of the filtrate was loaded onto a protamine chloride - POROS column (4.6 x 50 mm) equilibrated with buffer A. The flow rate was 10 ml/min. The column was washed with 10.0 ml of buffer A, and the adsorbed fraction was eluted with the same buffer containing NaCl by a stepwise increase of the NaCl concentration: 6.6 ml buffer A / 0.04 M NaCl; 6.6 ml buffer A / 0,06 M NaCl; 6.6 ml buffer A / 0.23 M NaCl followed by a linear gradient of 0.23 - 1.20 M NaCl (4.2 ml) in buffer A. Fractions of 6.0 ml were collected, assayed for XT activity and evaluated by SDS-PAGE. Cyclic chromatography was repeated 50 times. The purified enzyme was collected, concentrated to 1.0 ml using ultrafiltration techniques and stored at - 75 °C.

*Step 5: SDS-Polyacrylamide gel electrophoresis (SDS-PAGE) -* The protein composition of various fractions was estimated by SDS-PAGE. Briefly, 12.1 µl of sample was added to 4.7 µl of sample buffer (1.00 M Tris/HCl / 1.17 M sucrose, 0.28 M SDS, 2.08 mM EDTA, 0.88 mM Serva Blue G250, 0.70 mM phenol red, 0.10 M DTT, pH 8.5) and heated for 10 minutes at 99°C. After the sample had been loaded, SDS-polyacrylamide gel electrophoresis was carried out on a 4-12% bis-tris polyacrylamide gel with 3-(N-morpholino) propanesulfonic acid (MOPS) running buffer (1.00 M MOPS/Tris / 69.3 mM SDS, 20.5 mM EDTA, pH 7.7). Protein bands were detected by Coomassie Brilliant Blue or by silver staining. The Coomassie bands were excised and characterized by MALDI mass spectrometry and amino acid sequence analysis.

**Example 6:** *MALDI mass spectrometry.* Coomassie-stained proteins were excised from the gel, repeatedly washed with H₂O and H₂O/acetonitrile and digested overnight with trypsin and endoproteinase Lys-C at 37°C. The peptides generated in the supernatant were analyzed by MALDI mass spectrometry. Sample preparation was achieved following the thin film preparation techniques (13). Briefly, aliquots of 0.3 µl of a nitrocellulose containing saturated solution of a-cyano-4- hydroxycinnamic acid in acetone were deposited onto individual spots on the target. Subsequently, 0.8 µl 10% formic acid and 0.4 µl of the digest sample was loaded on top of the thin film spots and allowed to dry slowly at ambient temperature. To remove salts from the digestion buffer the spots were washed with 10% formic acid and with H₂O. MALDI mass spectra were recorded in the positive ion mode with delayed extraction on a Reflex II time-of-flight instrument equipped with a SCOUT multiprobe inlet and a 337 nm nitrogen laser. Ion acceleration voltage was set to 20.0 kV, the reflector voltage was set to 21.5 kV and the first extraction plate was set to 15.4 kV. Mass spectra were obtained by averaging 50-200 individual laser shots. Calibration of the spectra was performed internally by a two-point linear fit using the autolysis products of trypsin at m/z 842.50 and m/z 2211.10.

**Example 7:** *Amino acid sequence analysis of XT.* The MW 120.000 Coomassie-stained protein was excised from the gel, repeatedly washed with H₂O and H₂O/acetonitrile and digested with trypsin and endoproteinase Lys-C overnight. For HPLC separation the excised gel fragment was extracted twice with 0.1 % TFA/60% acetonitrile. The extracted enzymatic fragments were separated on a capillary HPLC system equipped with a 140B solvents delivery system (PE Biosystems), Acurate splitter (LC-Packings), UV absorbance detector 759A (PE Biosystems), U-Z capillary flow cell (LC-Packings) and Probot fraction collector (BAI) using reversed-phase column (Hypersil C18 BDS, 3 µm, 0.3 x 150 mm) and a linear gradient from 12% acetonitrile, 0.1 % TFA to 64% acetonitrile, 0.08% TFA in 90 min with a flow rate of 4 µl/min at RT. Peptide elution was monitored at 214 nm and individual fractions from the HPLC separation were analysed by MALDI mass spectrometry. Sequence analysis of separated fragments was performed on a Procise Protein Sequencer 494 cLC using standard programs supplied by PE Biosystems.

**Example 8:** *Determination of XT activity.* Determination of XT activity is based on the incorporation of [14C]-D-xylose into the recombinant bikunin according to a previously described method (Brinkmann et al., 1997, J. Biol. Chem., 272, 11171-11175). For analysis of the substrate specificity of the recombinant xylosyltransferases synthetic peptides containing the XT recognition sequence were used as acceptor. The reaction mixture for the assay contained, in a total volume of 100 µl: 50 µl of XT solution, 25 mM 4-morpholineethanesulfonic acid (pH 6.5), 25 mM KCl, 5 mM KF, 5 mM MgCl₂, 5 mM MnCl₂, 1.0 µM UDP-[¹⁴C]-D-xylose, and 1.5 µM of the synthetic peptides. After incubation for 75 min at 37°C, the reaction mixtures were placed on discs (25 mm diameter) of Immobilon-AV membrane, which immobilizes even small peptides by covalent links (Pfund & Bourdage, 1990, Mol. Immunol. 27, 495-502), and allowed to dry. It was then washed for 10 min with 10% trichloroacetic acid and three times with 5% trichloroacetic acid solution. Incorporated radioactivity was determined by liquid scintillation counting.

**Example 9:** *Antiserum Preparation.* The synthetic peptide CSRQKELLKRKLEQQEK deduced from the sequenced peptides 2 and 10 of the enzymatically cleaved XT was synthesized, purified by HPLC and used for immunization of rabbits (BioScience, Germany). Polyclonal antiserum was obtained by injection of the above antigen followed **Example 10:**
*Preparation of solid-phase antigen*. The antigen CSRQKELLKRKLEQQEK was immobilized on POROS 20 EP. After 1.6 mg antigen was dissolved in 1.2 ml of 10 mM phosphate / 0.15 M NaCl, pH 7.4 0.60 ml of 100 mM phosphate / 1.50 M NaCl, pH 7.4 was added. 400 mg POROS 20 EP was suspended in the solution and the suspension was mixed gently on a shaker at RT. At 10-min intervals, five times in total, an additional 0.24 ml of 100 mM phosphate / 1.50 M NaCl, pH 7.4 was added to the suspension. After additional shaking for 5 days at RT the suspension was filtered on a sintered glass funnel. The matrix was suspended in 4 ml 0.2 M phosphate / 0.1 M 2-mercaptoethanol, pH 7.4 and mixed on a shaker for 2 h at RT. The matrix was washed in a sintered glass funnel using 20 ml of 10 mM phosphate / 0.15 M NaCl, pH 7.4 and 20 ml of 1.0 M NaCl. After additional washing with 20 ml of 10 mM phosphate, pH 7.4, the matrix was packed in a PEEK column (4.6 x 50 mm).

**Example 11:** *Antibody purification.* Antiserum was adjusted to 50 mM Tris/HCl, pH 8.0. The solution was clarified by passage through a 0.2 µm filter. 0.4 ml filtrate was applied at 10 ml/min to the antigen column previously equilibrated with buffer B (50 mM Tris/HCl, pH 8.0). After the column was washed with 4.1 ml buffer B and with 12.5 ml buffer B / 0.15 M NaCl, the adsorbed antibody was eluted using 12.4 ml of 50 mM sodium citrate / 0.15 M NaCl, pH 3.0 followed by 3.4 ml of 100 mM sodium citrate / 1.5 M NaCl, pH 3.0. The eluate was collected as 10 ml fractions in tubes containing 2 ml of 0.5 M Tris/HCl, pH 8.0, to immediately neutralize the citric acid.

**Example 12:** *Preparation of immunoaffinity column.* Purified antibody was concentrated to a protein concentration of 0.3 mg/ml using ultrafiltration with YM1 cellulose membranes. The antibody solution was adjusted to 10 mM phosphate / 0.15 M NaCl, pH 7.4. After filtration of the solution through a 0.2 µm filter 100 µl filtrate was applied at 0.2 ml/min to a POROS 20 PA column (2.1 x 30 mm). This step was repeated 17 times. Adsorbed antibody was crosslinked using cross-linking solution (100 mM triethanolamine, pH 8.5). After the column was washed with 5 ml of buffer C (10 mM phosphate / 0.15 M NaCl, pH 7.4) 2 ml cross-linking solution was applied at 0.5 ml/min onto the cartridge. The procedure was repeated 6 more times, using a total volume of 14 ml cross-linking solution. To block unreacted functional groups on the cross-linking reagents 2 ml of 100 mM monoethanolamine, pH 9.0 (quenching solution), was loaded onto the cartridge at 0.5 ml/min. The column was washed using 2 ml of buffer C and the cross-linking step was repeated using another 2 ml quenching solution. The immunoaffinity column was cycled between buffer C and 12 mM HCl / 0.15 M NaCl 3 times using a total volume of 12 ml of solution.

**Example 13:** *Immunoaffinity column purification of XT.* XT-containing fractions eluted from the heparin affinity matrix were desalted using diafiltration with YM1 cellulose membranes and passed through a 0.2 µm filter. 100 µl of this XT-sample was applied to the immunoaffinity column equilibrated with buffer D (20 mM Tris/HCl, pH 8.0) at a flow rate of 1 ml/min. The column was washed with 1.4 ml of buffer D and with 8.5 ml of buffer D / 0.15 M NaCl. The XT activity was eluted with 4.2 ml of 12 mM HCl followed by 1.2 ml of 12 mM HCl / 1.5 M NaCl. Alternatively the elution was performed using 100 µl of antigen at 1 mg/ml in buffer D. Fractions (1 ml) were collected into tubes containing 1 ml of 0.1 M Tris/HCl, pH 8.0. The XT activities of the fractions were determined.

**Example 14:** *Aprotinin affinity chromatography.* 200 µl of desalted XT solution from the heparin purification step was applied at 10 ml/min to the aprotinin column previously equilibrated with buffer A. After washing the column with 6.6 ml of buffer A the adsorbed protein was eluted stepwise using the same buffer containing 0.3 M NaCl (10.0 ml), 0.54 M NaCl (10.0 ml), 1.00 M NaCl (10.0 ml) and 1.50 M NaCl (2.4 ml). **Example 15:** *Western Blot Analysis.* For western blot analysis, proteins were transferred to polyvinylidene difluoride membrane in a semi-dry instrument (Novex). After transfer nonspecific antibody binding sites were blocked with 2% BSA in 0.1 M Tris/HCl, pH 7.2, for 1 h at RT. The membrane was incubated with antiserum in 50 mM phosphate / 0.15 M NaCl, 0.5 ml/l Tween 20, pH 7.4 at 1:1000 dilution for 1 h. Bound antibody was detected using a second anti-rabbit goat immunoglobulin coupled to horse-radish peroxidase at a 1:1000 dilution. The blot was developed using 4-chloro-1-naphthol.

**Example 16:** *Gel filtration chromatography.* A sample of 100 µl from the heparin purification step was applied at 1.0 ml/min to a TSK G3000 SW column (30 cm x 7.5 mm, 10 µm particle size) which had previously been equilibrated with buffer A / 0.15 M NaCl. Proteins were eluted with the same buffer. Fractions of 200 µl were collected and tested for XT activity. Column calibration was performed using thyroglobulin (669.000), ferritin (440.000), aldolase (158.000), albumin (67.000), ovalbumin (43.000).

**Example 17:** *N-glycosidase F digestion.* Aliquots (1 µg) of XT were digested with 3,1 x 10 ⁻³ units ofN-glycosidase F at 37°C for 1 h and 12 h (Table II). The samples were then subjected to SDS-PAGE, and protein bands were detected by silver staining.

**Example 18:** *Measurement ofprotein concentration.* Protein concentration was estimated by absorbance at 280 nm assuming E^{1%} _{1 cm} =10.0 or with the *Bicinchoninic Acid Protein Assay* using bovine serum albumin as a standard.

**Example 19:** *PCR-based cloning of human xylosyltransferase.* Degenerate oligonucleotide primers with deoxyinosine substitution were designed based upon the amino acid sequence of peptides obtained after digestion of the isolated human XT with trypsin or Lys-C. The first strand of cDNA was synthesized by the reverse transcription reaction using poly(A)⁺ RNA isolated from the chondrosarcoma cell line SW1353 as template and oligo(dT) as primer. The reverse transcription reaction was performed at 37°C for 2 h using 50 pmol of oligo(dT) primers, 1 µg of poly(A)⁺ RNA, a 0.5 mM concentration of each dNTP, 1x RT buffer and 200 units of RNase H deficient Moloney murine leukemia virus reverse transcriptase (Life Technologies, Eggenstein, Germany) in a final volume of 20 µl. For PCR amplification the reaction mixture contained 4 µl of the reverse transcription reaction solution, 50 pmol of each primer, a 0.25 mM concentration of each dNTP, 50 mM KCl, 10 mM Tris-HCl (pH 8.3), 1.5 mM MgCl₂ and 2.5 units of hot start *Taq* polymerase (Life Technologies) in a final volume of 50 µl. Amplification with degenerate oligonucleotide primers was carried out by 40 cycles at 94°C for 1 min, 48°C for 1 min, and 72°C for 2 min, followed by a final extension step at 72°C for 15 min. After agarose electrophoresis of the PCR products, DNA fragments were excised, subcloned into the pCR2.1 vector (Invitrogen. Groningen, Netherlands) and sequenced by the dideoxy chain termination method using *Taq* DNA polymerase (Big-dye terminator cycle sequencing kit, Perkin-Elmer, Norwalk, CT, USA) with an automated DNA sequencer (Applied Biosystems, Weiterstadt, Germany). Several clones were sequenced to compensate for misreading by *Taq* polymerase.

**Example 20:** *Rapid amplification of 5' and 3' cDNA ends (RACE).* For amplification of the 5' and 3' ends of the XT-I and XT-II isoform cDNA RACE experiments were performed using commercially available systems (Clontech, Heidelberg, Germany; Life Technologies) according to the manufacturers' instructions. For 3'-RACE 1 µg placenta poly(A)⁺ mRNA (Clontech) was reverse transcribed with a 3'-CDS primer (Clontech). PCR amplification of the 3' cDNA end of human XT-I was accomplished according to a touch-down PCR protocol (5 cycles: 94°C for 30 s, 72°C for 3 min, 5 cycles: 94°C for 30 s, 70°C for 45 s, 72°C for 3 min, 25 cycles: 94°C for 30 s, 65°C for 45 s, 72°C for 3 min) with the gene-specific primer GSP1b 5'-GTGGGTATGCAGAAGTGGGGGAAGGGAC-3' and the UPM primer mix (Clontech). An aliquot of the first PCR was subjected to semi-nested PCR (30 cycles) using the primer Con2111 5'-CCCTCCGCAATGCCTACA-3' and the UPM primer mix. The DNA fragments obtained were subcloned into the vector pCR2.1 (Invitrogen) and sequenced. PCR amplification of the 3' cDNA end of the XT-II isoform was carried out with the primers AB 10267 5'-ACTGAGGTCACGCAATACAA-3' and UPM using the touch-down PCR protocol. For 5'-RACE 1 µg of placenta poly(A)⁺ mRNA was subjected to a reverse transcription and 5' tailing reaction with the 5'-CDS primer and the SMART oligo (Clontech) according to the manufacturer's protocol. The 5' end of the XT-I cDNA was amplified using the primer GSP_776L 5'-GCCGCACTCAG-GTGATGAAGAAGT-3' and UPM with a touch-down PCR protocol. An aliquot was used as template in a second semi-nested PCR reaction with the primers GSP_503L 5'-ACCA-CCAGGACAAAGGCGATTCTGA-3' and UPM. The 5'-cDNA end of XT-II was obtained by 5'-RACE amplification using the primers AB10315L 5'-AGTCGAACAGTCCAGG-GCCC-3' and UPM mix. A new primer for a 5'-RACE reaction was designed based upon the nucleotide sequence of the largest fragment obtained (2 kbp), and the experiment was repeated with the primer AB5846L 5'-CACGATCTCGCACTTGGGGG-3' as described above. The nucleotide sequences of human XT-I and XT-II cDNA have been submitted to the GenBank/EBI Data Bank with the accession numbers AJ277441 and AJ277442.

**Example 21:** *Isolation of XT-I cDNA from human brain and chondrocyte CDNA libraries.* Plasmid-DNA was isolated from a human whole brain cDNA library (Life Technologies) and a human chondrocyte cDNA library (Clontech) and used as template in a PCR-based approach for isolation of the 5' end of the XT-I cDNA. Briefly, GSP_776L and 5'ADLD 5'-CTATTCGATGATGAAGATACCCCACCAAACCC-3' primers were used in a PCR reaction with 1 µg of plasmid DNA as template. The DNA fragments were subjected to agarose gel electrophoresis and 0.7-3 kb long fragments were excised from the gel and used as a template in a semi-nested PCR reaction with GSP_503L and 5'-ADLD primers. DNA fragments were then subcloned into the vector pCR2.1 and sequenced.

**Example 22:** *Cloning of rat XT-I and XT-II cDNA.* A RT-PCR based approach using primers based upon the nucleotide sequence of human XT-I and XT-II was employed for amplification of XT-I and XT-II cDNA from rat. The first strand ofcDNA was synthesized by the reverse transcription reaction using poly(A)⁺ RNA isolated from the rat liver cell line BRL3A as template and oligo(dT) as primer. DNA fragments obtained after PCR amplification using moderate stringent conditions were subcloned into the vector pCR2.1 and sequenced. The 5' and 3' ends of the XT-I and XT-II cDNA from rat tissue were amplified using the RACE strategy with gene-specific primers as described above. The nucleotide sequences of rat XT-I and XT-II cDNA have been submitted to the GenBank/EBI Data Bank with the accession numbers AJ295748 and AJ295749.

**Example 23:** *Expression levels of the XT-I and the XT-II isoform in human tissues.* A Human Multiple Tissue cDNA Panel (Clontech) was used for the analysis of expression levels. Levels of amplification of the housekeeping gene glyceraldehyde-3-phosphate dehydrogenase, whose transcript is always present in the tissues at an almost constant level, were determined in parallel for quality control. For amplification of the XT-I encoding transcript the primers 128U and 601L were used, whereas primers AB10267U and AB12394L 5'-GGAAGAGCTGGGTGTGGAAT-3' were employed for the XT-II. PCR reactions were carried out by 22-36 cycles at 94°C for 30 s, 60-65°C for 45 s and 72°C for 2 min. Amplification of a transcript was performed using a serial number of cycles to find the conditions for semiquantitative amplification, and aliquots were analyzed by agarose gel electrophoresis.

**Example 24:** *Transfection and transient expression of XT.* For construction of a eukaryotic expression vector a DNA fragment including the known coding sequence of XT-I cDNA was amplified by PCR using XT_ExplL 5'-TTTCCCGTTGAGATCCTGCT-3' and XT_Exp3U 5'-ACAGACAGCAACAACGAGAA-3' as primers and placenta first-strand cDNA (Clontech) as template. The 2450 bp fragment obtained was cloned into the vector pcDNA4/HisMax-TOPO (Invitrogen) resulting in the fusion of XT-I to the *Xpress* epitope. The plasmid pCG227-XT-I was then transiently transfected into CHO-K1 cells. The coding region of the XT-II cDNA was amplified by PCR using the primers AB_Exp9U 5'-AAAGGAAGGCAGAGGAAGC-3' and AB_Exp3L 5'-ACCCCTCCACTGT-CTGTAAG-3' and placenta first-strand cDNA as template. The obtained 2440 bp DNA fragment was cloned into the expression vector pcDNA4/HisMax-TOPO resulting in the fusion of XT-II to the aminoterminal *Xpress* epitope. The plasmid termed pCG226-XT-II was transiently transfected into CHO-K1 cells. 2x10⁵ cells precultured for 1 day in a 35 mm diameter cell culture dish were transfected with 2 µg of plasmid DNA and 6 µl of Fugene 6 transfection reagent (Roche, Mannheim, Germany). For determination of transfection efficiency CHO-K1 cells were transfected with 2 µg of the control plasmid pcDNA4/HisMax-TOPO-*lac*Z. 48 h after transfection the cell culture medium was harvested. Protein G agarose beads (Sigma, Deisenhofen, Germany) and mouse anti-*Xpress* monoclonal antibody (Invitrogen) were added to the cell culture supernatant and incubated at 4°C for 1 h. After centrifugation at 10.000 g for 1 min the absorbed proteins were twice washed with PBS and resuspended in a final volume of 50 µl. XT activity was then assayed in the samples.

**Example 25:** *Characterization of the reaction products.* For characterization of the reaction product of recombinant XT-I the peptide QEEEGSGGGQK, which is homologous to the amino terminus of bikunin (Brinkmann *et al*., 1997, 1.c.), was used as acceptor in the XT activity assay. After incubation for 75 min at 37°C the enzyme was heat-inactivated by incubation for 15 min at 65°C and the reaction mixture was used for α- and β-xylosidase treatment and alkaline β-elimination. For the linkage-specif digestion of the bound [¹⁴C]-D-xylose 4 mU of α-xylosidase (Seikagaku Corporation, Tokyo, Japan) or 4 mU of β-xylosidase (Sigma, Dreieich, Germany) were added to the samples and incubated for 60 min at 37°C. The reaction mixtures were then placed on Immobilon-AV membrane discs and allowed to dry. The discs were washed with trichloroacetic acid as described above and the remaining incorporated radioactivity was determined against appropriate controls. The alkaline cleavage of the O-glycosidic linkage between the [¹⁴C]-D-xylose and the β-hydroxyamino acid serine was performed as described elsewhere (Montreuil *et al*., 1994). Briefly, the reaction mixture was adjusted to pH 10 with diluted NaOH and an equal volume of cold, freshly prepared sodiumboro-hydride solution (2 M sodiumborohydride in 0.1 M NaOH) and was added. After incubation at 45°C for 16 h the cooled solution was neutralized by adding 50% acetic acid and placed on Immobilon-AV membrane discs. After drying the discs were washed with trichloroacetic acid and the remaining radioactivity was measured by liquid scintillation counting.

## Claims

1. An isolated protein having the biological activity of human UDP-D-xylose:proteoglycan core protein β-D-xylosyltransferase (hXT), being an isoform thereof and designated as
(i) hXT-I, represented by the amino acid sequence of SEQ ID No. 2, or
(ii) hXT-II, represented by the amino acid sequence of SEQ ID No. 4.

2. hXT-I of claim 1 having a molecular mass of 91.000 (91 kD).

3. hXT-II of claim 1 having a molecular mass of 97.000 (97 kD).

4. A DNA sequence coding for a protein of claim 1.

5. A DNA sequence of claim 4 comprising the nucleotide sequence of SEQ ID No. 1.or SEQ ID No. 3

6. A process for manufacturing recombinant human xylosyltransferase isoenzym I or II (hXT-1 I / hXT-II) as specified in any of the claims 1 - 3, the process comprising the following steps:
(i) culturing human tissue cells having an enhanced activity of hXT by adapting the cells to hollow fiber culture conditions in a serum-free medium,
(ii) purifying and isolating hXT with a molecular weight of 120.000 (SDS-PAGE) from the cell supernatant obtained according to step (i) by heparin affinity chromatography and protamine chloride or aprotinin affinity chromatography,
(iii) enzymatically digesting purified hXT of step (ii) and designing primers from the separated proteolytic fragments,
(iv) PCR-based cloning of hXT-1 or hXT-II cDNA using proteolytic fragments of step (iii), and
(v) expressing recombinant hXT-1 or hXT-II protein using an expression vector comprising the DNA sequence of hXT-I or hXT-II.

7. A process according to claim 6, wherein the human tissue cells are JAR choriocarcinoma cells (ATCC HTB-144).

8. A pharmaceutical composition comprising a protein of claim 1, 2 or 3 and a pharmaceutically acceptable carrier, diluent or excipient.

9. Use of a protein of claim 1, 2 or 3 for the manufacture of a medicament for the treatment of sclerotic diseases and chronic inflammatory joint diseases.

10. Use of claim 9, wherein said medicament is an inhibitor or antagonist of said protein.

11. Use of a protein of claim 1, 2 or 3 for the manufacture of a product suitable as diagnostic marker for evaluating pathological conditions.

12. Use of a DNA molecule of claim 4 or 5 for the manufacture of a product suitable as gene marker.

13. A protein of claim 1, 2 or 3 for use as a medicament for the treatment of sclerotic diseases and chronic inflammatory joint diseases.

## Patentansprüche

1. Isoliertes Protein mit der biologischen Aktivität von humaner UDP-D-Xylose: Proteoglycan Kern-Protein β-D-Xylosyltransferase (hXT), welches eine Isoform derselben ist und bezeichnet ist als
(i) hXT-I, repräsentiert durch die Aminosäure-Sequenz gemäß SEQ ID No. 2, oder
(ii) hXT-II, repräsentiert durch die Aminosäure-Sequenz gemäß SEQ ID No. 4.

2. hXT-I nach Anspruch 1 mit einer Molekularmasse von 91.000 (91 kD).

3. hXT-II nach Anspruch 1 mit einer Molekularmasse von 97.000 (97 kD).

4. DNA Sequenz, welche für eine Protein gemäß Anspruch 1 kodiert.

5. DNA Sequenz nach Anspruch 4, welche eine Nukleotid-Sequenz gemäß SEQ ID No. 1 oder gemäß SEQ ID No. 3 umfasst.

6. Verfahren zur Herstellung von rekombinantem Xylosyltransferase-Isoenzym I oder II (hXT-I / hXT-II) gemäß einem der Ansprüche 1 - 3, umfassend die folgenden Schritte:
(i) Kultivierung von humanen Gewebezellen, die eine gesteigerte Aktivität von hXT besitzen, durch Anpassung der Zellen an Holhlfaser-Kulturbedingungen in einem serumfreien Medium,
(ii) Reinigung und Isolierung von hXT mit einem Molekulargewicht von 120.000 (SDS-PAGE) aus dem nach Schritt (i) gewonnenen Überstand durch Heparin-Affinitätschromatographie und Protaminchlorid- oder Aprotinin-Affinitätschromatographie,
(iii) enzymatische Spaltung von nach Schritt (ii) gereinigter hXT und Entwerfen von Primern aus den abgetrennten proteolytischen Fragmenten,
(iv) PCR- basierendes Klonieren von hXT-I oder hXT-II cDNA unter Verwendung der proteolytischen Fragmente des Schrittes (iii), und
(v) Exprimieren von rekombinantem hXT-I oder hXT-II Protein unter Verwendung eines Expressionsvektors, welcher die DNA Sequenz von hXT-I oder hXT-II umfasst.

7. Verfahren nach Anspruch 6, wobei die humanen Gewebezellen JAR Choriocarcinoma-Zellen (ATCC HTB-144) sind.

8. Pharmazeutische Zubereitung umfassend ein Protein nach Anspruch 1, 2 oder 3 und ein pharmazeutisch akzeptierbarer Träger, Verdünner oder Hilfsstoff.

9. Verwendung eines Proteins nach Anspruch 1, 2 oder 3 zur Herstellung eines Medikamentes zur Behandlung von sklerotischen Krankheiten und chronischen entzündlichen Gelenkkrankheiten.

10. Verwendung nach Anspruch 9, wobei besagtes Medikament ein Inhibitor oder ein Antagonist des besagten Proteins ist.

11. Verwendung eines Proteins nach Anspruch 1, 2 oder 3 zur Herstellung eines Produktes, das als diagnostischer Marker zur Bewertung pathologischer Zustände geeignet ist.

12. Verwendung eines DNA-Moleküls gemäß Anspruch 4 oder 5 zur Herstellung eines Produktes, das als Genmarker geeignet ist.

13. Ein Protein nach Anspruch 1, 2 oder 3 zur medikamentösen Anwendung bei der Behandlung von sklerotischen Krankheiten und chronischen entzündlichen Gelenkkrankheiten.

## Revendications

1. Protéine isolée ayant l'activité biologique de la UDP-D-xylose:protéine centrale de protéoglycane β-D-xylosyltransférase humaine (hXT), étant une isoforme de celle-ci et désignée par
(i) hXT-I, représentée par la séquence d'acides aminés SEQ ID n°2, ou
(ii) hXT-II, représentée par la séquence d'acides aminés SEQ ID n°4.

2. hXT-I selon la revendication 1, ayant une masse moléculaire de 91 000 (91 kD).

3. hXT-II selon la revendication 1, ayant une masse moléculaire de 97 000 (97 kD).

4. Séquence d'ADN codant pour une protéine selon la revendication 1.

5. Séquence d'ADN selon la revendication 4, comprenant la séquence nucléotidique de SEQ ID n°1 ou de SEQ ID n°3.

6. Procédé de production d'une isoenzyme de xylosyltransférase humaine I ou II (hXT-I / hXT-II) recombinée telle que spécifiée selon l'une quelconque des revendications 1-3, le procédé comprenant les étapes suivantes :
(i) la culture des cellules de tissu humain ayant une activité accrue de hXT par l'adaptation des cellules à des conditions de culture sur fibres creuses dans un milieu dépourvu de sérum,
(ii) la purification et l'isolement de la hXT ayant un poids moléculaire de 120 000 (SDS-PAGE) à partir du surnageant cellulaire obtenu selon l'étape (i) par chromatographie d'affinité à l'héparine et chromatographie d'affinité à l'aprotinine ou au chlorure de protamine,
(iii) la digestion enzymatique de la hXT purifiée de l'étape (ii) et la conception des amorces à partir des fragments protéolytiques séparés,
(iv) le clonage par PCR de l'ADNc de hXT-I ou de hXT-II à l'aide des fragments protéolytiques de l'étape (iii), et
(v) l'expression de la protéine hXT-I ou hXT-II recombinée à l'aide d'un vecteur d'expression comprenant la séquence d'ADN de hXT-I ou hXT-II.

7. Procédé selon la revendication 6, dans lequel les cellules de tissu humain sont des cellules de choriocarcinome JAR (ATCC HTB-144).

8. Composition pharmaceutique comprenant une protéine selon la revendication 1, 2 ou 3 et un véhicule, diluant ou excipient pharmaceutiquement acceptable.

9. Utilisation d'une protéine selon la revendication 1, 2 ou 3, pour la fabrication d'un médicament destiné au traitement de maladies sclérotiques et de maladies articulaires inflammatoires chroniques.

10. Utilisation selon la revendication 9, dans laquelle le médicament est un inhibiteur ou antagoniste de ladite protéine.

11. Utilisation d'une protéine selon la revendication 1, 2 ou 3, pour la fabrication d'un produit convenable comme marqueur de diagnostic pour l'évaluation de conditions pathologiques.

12. Utilisation d'une molécule d'ADN selon la revendication 4 ou 5, pour la fabrication d'un produit convenable comme marqueur génétique.

13. Protéine selon la revendication 1, 2 ou 3, pour une utilisation comme médicament destiné au traitement de maladies sclérotiques et de maladies articulaires inflammatoires chroniques.
